# EUROPEAN PATENT APPLICATION

(11) **EP 4 386 006 A1**
(43) Date of publication of application: **19.06.2024**
(21) Application number: 22836843.7
(22) Date of filing: 04.07.2022
(51) Int. Cl.: C07K 16/46, A61K 39/395, A61P 35/00

(54) **ANTIBODY-DRUG CONJUGATE AND APPLICATION THEREOF**

(30) Priority: 05.07.2021 CN 202110757803
(71) Applicant: Jiangsu Alphamab Biopharmaceuticals Co., Ltd., Jiangsu 215000 (CN)
(72) Inventor: XU, Ting, Suzhou, Jiangsu 215000 (CN); WANG, Pilin, Suzhou, Jiangsu 215000 (CN); GUO, Kangping, Suzhou, Jiangsu 215000 (CN); ZHAO, Yonghao, Suzhou, Jiangsu 215000 (CN); PENG, Jianjian, Suzhou, Jiangsu 215000 (CN)
(74) Representative: Latscha Schöllhorn Partner AG
(86) International application number: PCT/CN2022/103592
(87) International publication number: WO 2023/280092

(57) **Abstract**

An antibody-drug conjugate, comprising a bispecific antibody targeting HER2 or an antigen-binding fragment thereof. Also provided are a preparation method for and an application of the antibody-drug conjugate, and a pharmaceutical composition comprising the antibody-drug conjugate. The antibody-drug conjugate can effectively kill tumors.

## Description

### TECHNICAL FIELD

The present application relates to the field of biomedicine and particularly to an antibody-drug conjugate and use thereof.

### BACKGROUND

Members of the HER receptor tyrosine kinase family are important mediators of cell growth, differentiation and survival. This receptor family includes four distinct members, including epidermal growth factor receptor (EGFR, ErbB1 or HER1), HER2 (ErbB2 or p185neu), HER3 (ErbB3) and HER4 (ErbB4 or tyro2). Members of this receptor family have been implicated in various types of human malignancies.

Bispecific antibodies (BsAbs) are immunoglobulin molecules with two different ligand-binding sites. They have two different Fab sequences instead of identical sequences in two Fab arms of classical antibody, so the two arms of the Y-shaped structure can bind to different antigenic epitopes. The application of bispecific antibodies in cancer therapy has been reviewed in several articles (Carter 2001; Chames and Baty 2009; and Chames and Baty 2009). Antibody-drug conjugates obtained by linking a cytotoxic drug to an antibody that binds to an antigen that is expressed on the surface of cancer cells and can be internalized by cells can selectively deliver the drug to the cancer cells and is thus expected to cause accumulation of the drug within the cancer cells and to kill the cancer cells.

### SUMMARY

The present application provides an antibody-drug conjugate, comprising a HER2-targeting bispecific antibody or an antigen-binding fragment thereof. The antibody-drug conjugate can specifically bind to at least one (e.g., at least 2) epitopes in human HER2. The antibody-drug conjugate of the present application may have the following properties: (1) effectively killing tumor cells, wherein the antibody moiety and the drug moiety can synergistically kill tumors; (2) specifically binding to tumor cells; (3) having good stability in serum; (4) having a stronger bystander killing effect on tumor cells; (5) having a good ADCC effect; and/or (6) having relatively good endocytosis efficiency. The present application also provides a preparation method for the antibody-drug conjugate, a composition comprising the antibody-drug conjugate, and use of the antibody-drug conjugate and the composition.

In one aspect, the present application provides an antibody-drug conjugate, comprising a HER2-targeting bispecific antibody or an antigen-binding fragment thereof.

In certain embodiments, the HER2-targeting bispecific antibody or the antigen-binding fragment thereof specifically binds to at least one epitope of human HER2.

In certain embodiments, the HER2-targeting bispecific antibody or the antigen-binding fragment thereof specifically binds to extracellular domain II of human HER2 and/or extracellular domain IV of human HER2.

In certain embodiments, the HER2-targeting bispecific antibody or the antigen-binding fragment thereof comprises a first light chain and a second light chain.

In certain embodiments, the first light chain comprises first LCDR1-3, wherein the first LCDR1 comprises an amino acid sequence set forth in SEQ ID NO: 4.

In certain embodiments, the first LCDR2 comprises an amino acid sequence set forth in SEQ ID NO: 5.

In certain embodiments, the first LCDR3 comprises an amino acid sequence set forth in SEQ ID NO: 6.

In certain embodiments, the first light chain is capable of binding to the heavy chain of pertuzumab.

In certain embodiments, the second light chain comprises second LCDR1-3, wherein the second LCDR1 comprises an amino acid sequence set forth in SEQ ID NO: 1.

In certain embodiments, the second LCDR2 comprises an amino acid sequence set forth in SEQ ID NO: 2.

In certain embodiments, the second LCDR3 comprises an amino acid sequence set forth in SEQ ID NO: 3.

In certain embodiments, the second light chain is capable of binding to the heavy chain of trastuzumab.

In certain embodiments, the variable region of the first light chain and the second light chain comprises an amino acid sequence set forth in any one of SEQ ID NOs: 7-12.

In certain embodiments, the variable region of the first light chain and the second light chain comprises an amino acid sequence set forth in SEQ ID NO: 7.

In certain embodiments, the first light chain and the second light chain comprise an amino acid sequence set forth in any one of SEQ ID NOs: 13-18.

In certain embodiments, the first light chain is selected from the group consisting of: the light chain of pertuzumab or a mutant thereof and the light chain of trastuzumab or a mutant thereof; and/or, the second light chain is selected from the group consisting of: the light chain of pertuzumab or a mutant thereof and the light chain of trastuzumab or a mutant thereof.

In certain embodiments, amino acid sequences of the first light chain and the second light chain are identical.

In certain embodiments, the first light chain and the second light chain comprise an amino acid sequence set forth in SEQ ID NO: 13.

In certain embodiments, the HER2-targeting bispecific antibody or the antigen-binding fragment thereof comprises a first heavy chain and a second heavy chain, wherein the first heavy chain is capable of properly binding to the first light chain under physiological conditions or in an *in vitro* protein expression state.

In certain embodiments, the second heavy chain is capable of properly binding to the second light chain under physiological conditions or in an *in vitro* protein expression state.

In certain embodiments, the first heavy chain comprises a first heavy chain variable region, and the first heavy chain variable region is a heavy chain variable region of pertuzumab.

In certain embodiments, the second heavy chain comprises a second heavy chain variable region, and the second heavy chain variable region is a heavy chain variable region of trastuzumab.

In certain embodiments, the first heavy chain and the second heavy chain comprise heavy chain constant regions, wherein the heavy chain constant regions are derived from the constant region of a human IgG.

In certain embodiments, the Fc fragment of the first heavy chain and the second heavy chain comprises an amino acid sequence set forth in any one of SEQ ID NOs: 25-57.

In certain embodiments, the first heavy chain comprises an amino acid sequence set forth in 21 or 23.

In certain embodiments, the second heavy chain comprises an amino acid sequence set forth in 22 or 24.

In certain embodiments, the antibody-drug conjugate comprises a structure represented by formula 1:

M-(L1)ₐ-(L2)_{b}-D (formula 1),

wherein M represents the HER2-targeting bispecific antibody or the antigen-binding fragment thereof of the present application; L1 represents a linker linking to M, and L2 represents a linker linking to D; a and b are each independently selected from 0-10; D represents a drug.

In certain embodiments, the L1 and/or L2 are/is selected from the group consisting of: a cleavable linker, a non-cleavable linker, a hydrophilic linker, a hydrophobic linker, a charged linker, an uncharged linker, and a dicarboxylic acid-based linker.

In certain embodiments, the L1 and the M are linked by a sulfhydryl, azido or amide group on the M.

In certain embodiments, the M comprises a first heavy chain and a second heavy chain, and the first heavy chain and/or the second heavy chain comprise(s) a linking site capable of linking to the L1.

In certain embodiments, the linking site comprises a group capable of linking to the L1 following deglycosylation modification.

In certain embodiments, the group is located at the side group of the amino acid Q at position 297 of the first heavy chain; and/or is located at the side group of the amino acid Q at position 298 of the second heavy chain.

In certain embodiments, the group includes the amide group.

In certain embodiments, the linking site comprises a group capable of linking to the L1 following glycosylation modification.

In certain embodiments, the group is located at the side group of the amino acid N at position 299 of the first heavy chain; and/or is located at the side group of the amino acid N at position 300 of the second heavy chain.

In certain embodiments, the group comprises -N₃.

In certain embodiments, the glycosylation modification comprises: the M having been contacted with UDP-GalNAz, β-1,4-galactosyltransferase or a variant thereof.

In certain embodiments, the L 1 is capable of taking part in a SPAAC reaction.

In certain embodiments, the L1 is selected from the group consisting of: maleimide, succinimide-3-yl-N, and DBCO.

In certain embodiments, the L1 is DBCO-(PEG)ₙ₁, wherein ₙ₁ is an integer from 0-10, or the L1 is maleimide.

In certain embodiments, the L2 is selected from the group consisting of: a polypeptide, VC-PAB, N-succinimidyl-3-(2-pyridyldithio)propionate (SPDP), N-succinimidyl 4-(2-pyridyldithio)valerate (SPP), N-succinimidyl 4-(2-pyridyldithio)butyrate (SPDB), N-succinimidyl-4-(2-pyridyldithio)-2-sulfobutyrate (sulfo-SPDB), N-succinimidyl iodoacetate (SIA), N-succinimidyl (4-iodoacetyl)aminobenzoate (SIAB), maleimide PEG NHS, N-succinimidyl 4-(maleimidomethyl)cyclohexylcarboxylate (SMCC), sulfosuccinimidyl 4-(N-maleimidomethyl)cyclohexane-1-carboxylate (sulfo-SMCC) and 2,5-dioxopyrrolidin-1-yl 17-(2,5-dioxo-2,5-dihydro-1H-pyrrol-1-yl)-5,8,11,14-tetraoxy-4,7,10,13-tetraazaoctadecan-1-o ate (CX1-1).

In certain embodiments, the L2 is GGFG.

In certain embodiments, the drug has the ability to kill tumor cells, and/or the ability to inhibit tumor cell growth.

In certain embodiments, the drug includes small-molecule drugs.

In certain embodiments, the drug is selected from the group consisting of: a V-ATPase inhibitor, a Bcl2 inhibitor, an MCL1 inhibitor, an HSP90 inhibitor, an IAP inhibitor, an mTor inhibitor, a microtubule stabilizer, a microtubule destabilizer, auristatin, dolastatin, a maytansinoid, MetAP (methionine aminopeptidase), an inhibitor of nuclear export of protein CRM1, a DPPIV inhibitor, a proteasome inhibitor, an inhibitor of phosphoryl transfer reactions in mitochondria, a protein synthesis inhibitor, a CDK2 inhibitor, a CDK9 inhibitor, a kinesin inhibitor, an HDAC inhibitor, a DNA damaging agent, a DNA alkylating agent, a DNA intercalator, a DNA minor groove binder, a DHFR inhibitor, a nucleoside analog, an HD AC inhibitor, an anthracycline, a NAMPT inhibitor, SN-38 glucuronic acid, etoposide phosphate, nitrogen mustard, a proteosome inhibitor, a cytokine, and a Toll-like receptor agonist.

In certain embodiments, the drug is selected from the group consisting of: DM1, exatecan, DXd, MMAE, SN-38, calicheamicin, anthracyclin-5G, DM4, microtubule inhibitor SHR153024, PNU-159682, Duo5 toxin, an SN38 derivative or derivatives thereof.

In certain embodiments, the antibody-drug conjugate has a structure selected from the group consisting of: 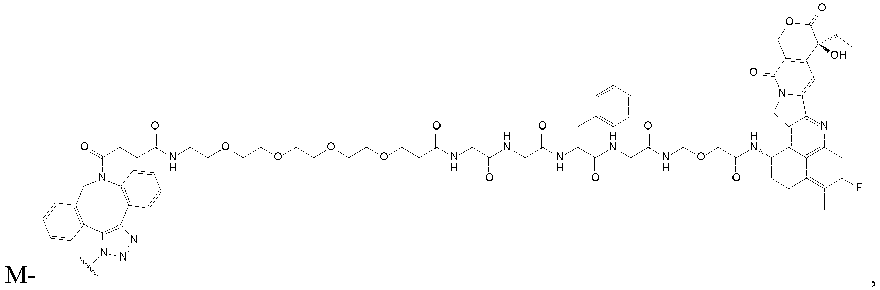 and 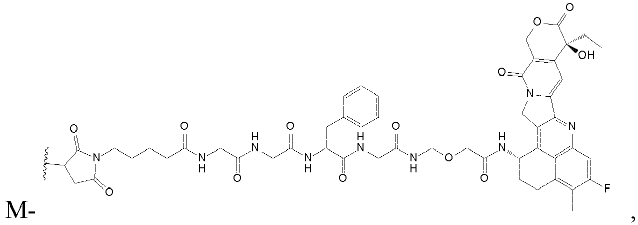 and

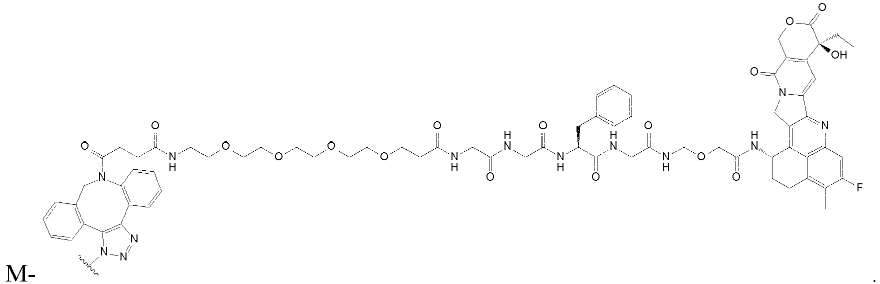

In certain embodiments, the antibody-drug conjugate has a drug/antibody ratio of about 2-6.

In another aspect, the present application provides a compound for preparing the antibody-drug conjugate of the present application, which has a structure represented by formula 2: M-(L1)ₐ (formula 2), wherein M represents the HER2-targeting bispecific antibody or the antigen-binding fragment thereof of the present application; L1 represents a linker linking to M; a is selected from 0-10.

In certain embodiments, the L1 is selected from the group consisting of: a cleavable linker, a non-cleavable linker, a hydrophilic linker, a hydrophobic linker, a charged linker, an uncharged linker, and a dicarboxylic acid-based linker.

In certain embodiments, the L1 and the M are linked by a sulfhydryl, azido or amide group on the M.

In certain embodiments, the M comprises a first heavy chain and a second heavy chain, and the first heavy chain and/or the second heavy chain comprise(s) a linking site capable of linking to the L1.

In certain embodiments, the linking site comprises a group capable of linking to the L1 following deglycosylation modification.

In certain embodiments, the group is located at the side group of the amino acid Q at position 297 of the first heavy chain; and/or is located at the side group of the amino acid Q at position 298 of the second heavy chain.

In certain embodiments, the group includes the amide group.

In certain embodiments, the linking site comprises a group capable of linking to the L1 following glycosylation modification.

In certain embodiments, the group is located at the side group of the amino acid N at position 299 of the first heavy chain; and/or is located at the side group of the amino acid N at position 300 of the second heavy chain.

In certain embodiments, the group comprises -N₃.

In certain embodiments, the glycosylation modification comprises: the M having been contacted with UDP-GalNAz, β-1,4-galactosyltransferase or a variant thereof.

In certain embodiments, the L 1 is capable of taking part in a SPAAC reaction.

In certain embodiments, the L1 is selected from the group consisting of: maleimide, succinimide-3-yl-N, and DBCO.

In certain embodiments, the L1 is DBCO-(PEG)ₙ₁, wherein n1 is an integer from 0-10, or the L1 is maleimide.

In certain embodiments, the compound comprises a structure selected from the group consisting of:

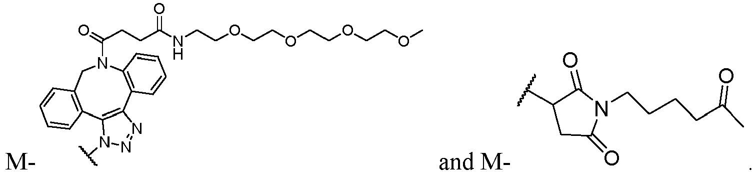

In another aspect, the present application provides a method for preparing the antibody-drug conjugate of the present application, comprising the following step: contacting the compound of the present application with the drug of the present application.

In another aspect, the present application provides a pharmaceutical composition, comprising the antibody-drug conjugate of the present application, or a pharmaceutically acceptable carrier. In another aspect, the present application provides a method for modulating the tumor microenvironment in a subject, comprising the following step: administering to the subject the antibody-drug conjugate of the present application, or the pharmaceutical composition of the present application.

In another aspect, the present application provides a method for modulating the immune response in a subject, comprising the following step: administering to the subject the antibody-drug conjugate of the present application, or the pharmaceutical composition of the present application.

In another aspect, the present application provides use of the antibody-drug conjugate of the present application or the pharmaceutical composition of the present application in the preparation of a medicament, wherein the medicament can prevent and/or treat tumors.

In certain embodiments, the tumors include solid tumors and/or non-solid tumors.

Other aspects and advantages of the present application will be readily apparent to those skilled in the art from the following detailed description. Only exemplary embodiments of the present application are shown and described in the following detailed description. As those skilled in the art will recognize, the content of the present application enables those skilled in the art to make changes to the specific embodiments disclosed without departing from the spirit and scope of the invention involved in the present application. Accordingly, descriptions in the drawings and specification are only illustrative rather than restrictive.

### BRIEF DESCRIPTION OF THE DRAWINGS

Specific features of the invention involved in the present application are set forth in appended claims. Features and advantages of the invention involved in the present application will be better understood by reference to the exemplary embodiments and drawings described in detail below. A brief description of the drawings is as below.
FIG. 1 shows the reaction for the glycosylation modification of the HER2-targeting bispecific antibody of the present application.
FIG. 2 shows an HPLC chromatogram of the glycosylated HER2-targeting bispecific antibody of the present application.
FIG. 3 shows the reaction for obtaining the antibody-drug conjugate of the present application.
FIG. 4 shows an HPLC chromatogram of the antibody-drug conjugate of the present application.
FIG. 5 shows the reaction for obtaining the antibody-drug conjugate of the present application.
FIG. 6 shows the results of Waters Xevo G2-QTOF mass spectrometry analysis of the antibody-drug conjugate of the present application.
FIG. 7 shows the results of Waters Xevo G2-QTOF mass spectrometry analysis of the antibody-drug conjugate of the present application.
FIG. 8 shows the results of Waters Xevo G2-QTOF mass spectrometry analysis of the light chain portion of DS-8201.
FIG. 9 shows the results of Waters Xevo G2-QTOF mass spectrometry analysis of the heavy chain portion of DS-8201.
FIG. 10 shows the binding affinity of the HER2-targeting bispecific antibody of the present application for FcyRIIIa.
FIG. 11 shows the binding affinity of the antibody-drug conjugate of the present application for FcyRIIIa.
FIG. 12 shows the binding affinity of the HER2-targeting bispecific antibody of the present application for FcyRI.
FIG. 13 shows the binding affinity of the antibody-drug conjugate of the present application for FcyRI.
FIG. 14 shows the abilities of the antibody-drug conjugates of the present application to bind tumor cells.
FIG. 15 shows the abilities of the antibody-drug conjugates of the present application to kill tumor cells after 3 days of treatment.
FIG. 16 shows the abilities of the antibody-drug conjugates of the present application to kill tumor cells after 5 days of treatment.
FIG. 17 shows the abilities of the antibody-drug conjugates of the present application to kill tumor cells after 3 days of treatment.
FIG. 18 shows the results of the inhibition of SK-BR-3 cell proliferation in each experimental group.
FIG. 19 shows the results of the inhibition of MDA-MB-468 cell proliferation in each experimental group.
FIG. 20 shows the results of the ADCC activity analysis of the antibody-drug conjugates of the present application.
FIG. 21 shows steps for determining the ADCC activity of the antibody-drug conjugates of the present application using a PBMC system.
FIGs. 22-23 show the results of the determination of the ADCC activity of the antibody-drug conjugates of the present application using the PBMC system.
FIGs. 24-26 show the endocytosis of the antibody-drug conjugates of the present application in tumor cells.
FIG. 27 shows the endocytosis of the antibody-drug conjugates of the present application in tumor cells.
FIG. 28 shows the results of the pharmacokinetic study of the antibody-drug conjugates of the present application.
FIG. 29 shows the proportion of NCI-N87 positive cells after endocytosis induced by the antibody-drug conjugate of the present application.
FIG. 30 shows the tumor volume growth curve of each group of mice of a human gastric cancer PDX tumor model.
FIG. 31 shows tumor volume growth curves of mice of a human gastric cancer HER2 IHC-PDX tumor model.

### DETAILED DESCRIPTION

The embodiments of the invention in the present application are described below with reference to specific examples, and other advantages and effects of the present invention will be readily apparent to those skilled in the art from the disclosure of the present specification.

### Definitions of Terms

In the present application, the term "antibody-drug conjugate" refers generally to an ADC, i.e., a binding protein (e.g., an antibody or an antigen-binding fragment thereof) linked to one or more chemical drugs. The chemical drugs may be any therapeutic agents and/or cytotoxic agents. The antibody-drug conjugate may have anywhere from 1 to 8 drugs conjugated to the antibody; for example, it may include drug loaded species of 2, 4, 6 or 8. In the present application, the drug may include mitotic inhibitors, anti-tumor antibiotics, immunomodulating agents, vectors for gene therapy, alkylating agents, anti-angiogenic agents, anti-metabolites, boron-containing agents, chemoprotective agents, hormones, anti-hormone agents, corticosteroids, photoactive therapeutic agents, oligonucleotides, radionuclide agents, topoisomerase inhibitors, tyrosine kinase inhibitors and/or radiosensitizers.

In the present application, the term "drug/antibody ratio" or "DAR" refers generally to the number of drugs linked to the antibody of the ADC. The DAR of an ADC may range from 1 to 8, or higher loads (e.g., 10) are also possible. The range of the DAR can depend on the number of linking sites on the antibody. In the present application, the DAR may be the number of drugs loaded onto an individual antibody. The DAR may also be an average or mean DAR of a group of ADCs.

In the present application, the term "HER2" refers generally to human epidermal growth factor receptor 2 (SwissProt P04626). In the present application, the HER2 may also be referred to as rbB-2, NEU, HER-2 or CD340. The HER2 may include any variants, isoforms and species homologs of HER2 which are naturally expressed by cells, including tumor cells, or are expressed by cells transfected with the HER2 gene or cDNA.

In the present application, the term "extracellular domain" refers generally to the extracellular domains of human HER2 (SwissProt P04626), which may include four extracellular domains I, II, III and IV

In the present application, the term "antibody" refers generally to an immunoglobulin molecule consisting of two identical pairs of polypeptide chains (each pair has one "light" (L) chain and one "heavy" (H) chain). The light chains of antibodies can be classified as κ and λ light chains. The heavy chains can be classified as µ, δ, γ, α or ε, and the isotypes of antibodies are defined as IgM, IgD, IgG, IgA and IgE, respectively. Within the light and heavy chains, the variable and constant regions are joined by a "J" region of about 12 or more amino acids, and the heavy chains further comprise a "D" region of about 3 or more amino acids. Each heavy chain consists of a heavy chain variable region (VH) and a heavy chain constant region (CH). The heavy chain constant region consists of 3 domains (CH1, CH2 and CH3). Each light chain consists of a light chain variable region (VL) and a light chain constant region (CL). The light chain constant region consists of one domain, CL. The constant region of an antibody may mediate the binding of the immunoglobulin to the host tissue or factor, including various cells of the immune system (e.g., effector cells) and the first component (C1q) of the classical complement system. The VH and VL regions can also be subdivided into regions with high variability (known as complementarity determining regions (CDRs)) interspersed with more conserved regions known as framework regions (FRs). Each VH and VL consists of 3 CDRs and 4 FRs arranged from the amino-terminus to the carboxy-terminus in the following order: FR1, CDR1, FR2, CDR2, FR3, CDR3, FR4. The variable regions (VH and VL) of each heavy/light chain pair form an antibody binding site. The distribution of amino acids to regions or domains follows the definition of Kabat Sequences of Proteins of Immunological Interest (National Institutes of Health, Bethesda, Md. (1987 and 1991)), or Chothia & Lesk (1987) J. Mol. Biol. 196:901-917; Chothia et al. (1989) Nature 342:878-883. The term "antibody" is not limited by any particular antibody-producing method. For example, it includes, in particular, recombinant antibodies, monoclonal antibodies and polyclonal antibodies. Antibodies may be antibodies of different isotypes, e.g., IgG (e.g., IgG1, IgG2, IgG3 or IgG4 subtype), IgA1, IgA2, IgD, IgE or IgM antibodies.

In the present application, the term "antigen-binding portion" refers to one or more portions of a full-length antibody that retain(s) the ability to bind the same antigen to which the antibody binds (e.g., HER2) and competes with an intact antibody for specific binding to the antigen. See generally, Fundamental Immunology, Ch. 7 (Paul, W., ed., 2nd Edition, Raven Press, N.Y. (1989), which is incorporated herein by reference in its entirety for all purposes. Antigen-binding portions can be produced by recombinant DNA techniques or by enzymatic or chemical cleavage of intact antibodies. In some cases, antigen-binding portions include Fab, Fab', F(ab')2, Fd, Fv, dAb and complementarity determining region (CDR) fragments, single-chain antibodies (e.g., scFv), chimeric antibodies, diabodies, and polypeptides that comprise at least a portion of an antibody that is sufficient to confer specific antigen-binding ability on the polypeptides. The antigen-binding portions (e.g., the antibody fragments described above) of an antibody can be obtained from a given antibody (e.g., the monoclonal antibody 2E12) by conventional techniques known to those skilled in the art (e.g., recombinant DNA techniques or enzymatic or chemical cleavage methods), and the antigen-binding portions of the antibody are screened for specificity in the same manner as intact antibodies.

In the present application, the term "Fd fragment" means an antibody fragment consisting of VH and CH1 domains; the term "Fv fragment" means an antibody fragment consisting of VL and VH domains of a single arm of the antibody; the term "dAb fragment" means an antibody fragment consisting of a VH domain (Ward et al., Nature 341:544 546 (1989)); the term "Fab fragment" means an antibody fragment consisting of VL, VH, CL and CH1 domains; the term "F(ab')2 fragment" means an antibody fragment comprising two Fab fragments linked by a disulfide bridge on the hinge region.

In the present application, the term "antibody Fc" refers generally to a human immunoglobulin chain constant region defined based on papain cleavage of an antibody. The Fc may be the carboxy-terminus of an immunoglobulin heavy chain constant region or a portion thereof. For example, an immunoglobulin Fc region may comprise a combination of two or more of the heavy chain CH2, CH3 and CH4 domains with an immunoglobulin hinge region. Based on the amino acid sequence of the heavy chain constant region, immunoglobulins can be divided into different types. There are mainly 5 types of immunoglobulins: IgA, IgD, IgE, IgG and IgM, some of which can be further divided into subtypes (isotypes), such as IgG-l, IgG-2, IgG-3, IgG-4, IgA-l and IgA-2. Selecting specific immunoglobulin Fc regions from specific types and subtypes of immunoglobulins is within the knowledge of those skilled in the art. For example, the Fc may include at least one immunoglobulin hinge region, one CH2 domain and one CH3 domain, for example, a human IgG1 Fc.

In the present application, the term "bispecific antibody" refers generally to an antibody capable of binding to two antigens or antigenic epitopes. The bispecific antibody may comprise a light chain and a heavy chain of an antibody capable of specifically binding to a first antigen or antigenic epitope, and a light chain and a heavy chain of an antibody capable of specifically binding to a second antigen or antigenic epitope. In one embodiment of the present application, in the bispecific antibody, the light chain of the antibody capable of specifically binding to the first antigen or antigenic epitope and the light chain of the antibody capable of specifically binding to the second antigen or antigenic epitope have the same sequence. In one embodiment of the present application, the heavy chain of the antibody capable of specifically binding to the first antigen or antigenic epitope and the heavy chain of the antibody capable of specifically binding to the second antigen or antigenic epitope in the bispecific antibody have different sequences.

In the present application, the term "epitope" or "antigenic epitope" refers generally to a site on an antigen to which an immunoglobulin or antibody specifically binds. "Epitope" is also referred to in the art as "antigenic determinant". Epitopes or antigenic determinants usually consist of chemically active surface groups of molecules such as amino acids or carbohydrates or sugar side chains and usually have specific three-dimensional structural characteristics, as well as specific charge characteristics. For example, an epitope typically comprises at least 3, 4, 5, 6, 7, 8, 9, 10, 11, 12, 13, 14 or 15 contiguous or non-contiguous amino acids in a unique spatial conformation, and it may be "linear" or "conformational". See, e.g., Epitope Mapping Protocols in Methods in Molecular Biology, vol. 66, G. E. Morris, Ed. (1996). In a linear epitope, all points of interaction between a protein and an interacting molecule (e.g., an antibody) exist linearly along the primary amino acid sequence of the protein. In a conformational epitope, the points of interaction exist across amino acid residues on the protein that are separated from one another.

In the present application, the term "specific binding" refers generally to binding compared to non-specific adsorption. Examples of the criterion for determining whether the binding is specific or not can include dissociation constant (e.g., "KD"). For example, the KD value of the bispecific antibody or the antigen-binding fragment thereof for the HER2 protein is 1 × 10⁻⁵ M or less, 5 × 10⁻⁶ M or less, 2 × 10⁻⁶ M or less, or 1 × 10⁻⁶ M or less; or is 5 × 10⁻⁹ M or less, 2 × 10⁻⁹ M or less, or 1 × 10⁻⁹M or less. The binding can be measured by a known method such as surface plasmon resonance, ELISA, or RIA.

The term "CDR" as used herein refers to complementarity determining region (CDR). It is known that each of the heavy and light chains of an antibody molecule has 3 CDRs. The CDRs, also known as hypervariable domains, are present in the sites where the variability is very high of the primary structure in variable regions of the heavy and light chains of the antibody. There are 3 separate CDRs in the primary structure of the heavy and light polypeptide chains. In this specification, as for the CDRs of an antibody, the CDRs of the heavy chain are represented by CDRH1, CDRH2 and CDRH3 from the amino-terminal side of the amino acid sequence of the heavy chain, and the CDRs of the light chain are represented by CDRL1, CDRL2 and CDRL3 from the amino-terminal side of the amino acid sequence of the light chain. These sites are close to one another in steric structure and determine the specificity for the bound antigen.

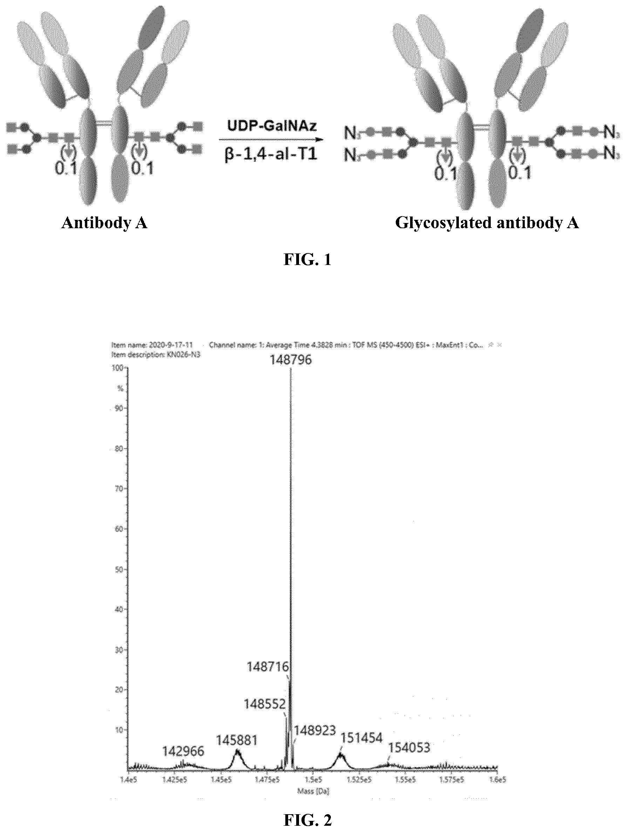

In the present application, the 20 conventional amino acids and their abbreviations follow conventional usage. See Immunology A Synthesis (2nd Edition, E. S. Golub and D. R. Gren, Eds., Sinauer Associates, Sunderland, Mass. (1991)), which is incorporated herein by reference. In the present application, the term "pertuzumab" refers generally to the antibody under the trade name Perjeta, also known as 2C4. The amino acid sequences of the light and heavy chain variable regions of pertuzumab can be found in FIG. 2 of US20090285837A1. Pertuzumab is a recombinant humanized monoclonal antibody that specifically binds to the extracellular dimerization domain (subdomain II) of epidermal growth factor receptor 2 (HER2). Pertuzumab binds to HER2 to block the heterodimerization of HER2 with other HER receptors, thereby slowing tumor growth. Pertuzumab can be used for treating HER2 positive metastatic breast cancer, and can be used for treating early breast cancer.

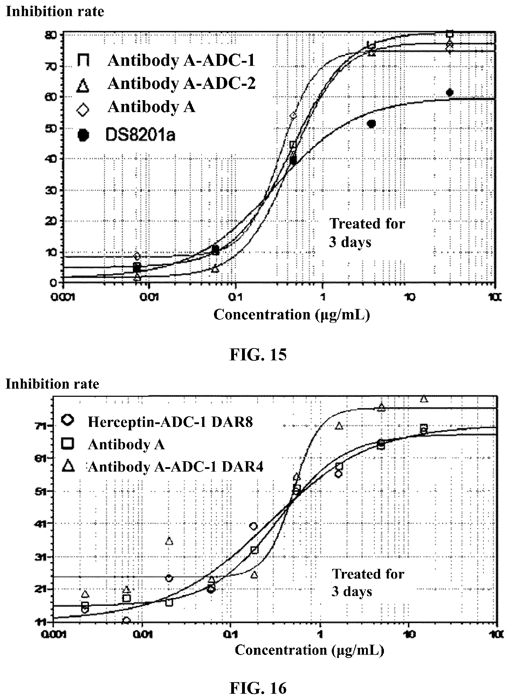

In the present application, the term "trastuzumab" refers generally to the antibody under the trade name Herceptin^{®}. The amino acid sequences of the light and heavy chains of trastuzumab can be found in FIG. 16 of US20090285837A1. Trastuzumab is a recombinant DNA-derived humanized monoclonal antibody that can be produced by mammalian cells (Chinese hamster ovary (CHO) cells) cultured in suspension in a sterile medium. Trastuzumab can specifically bind to the extracellular domain of HER2 and can also stimulate the body's immune cells to destroy tumor cells. Trastuzumab can be used for treating HER2 positive metastatic breast cancer, early breast cancer and HER2 positive metastatic gastric adenocarcinoma or gastroesophageal junction adenocarcinoma.

In the present application, the term "exatecan" refers generally to DX-8951, which is a DNA topoisomerase I inhibitor under CAS No. 171335-80-1. DXd is a derivative of exatecan (DX-8951) under CAS No. 1599440-33-1. As medicinal camptothecin derivatives, exatecan and DXd are moderately toxic drugs, and the use of such drugs in ADCs can reduce the toxic side effects caused by toxin shedding.

In the present application, the term "linker" refers generally to a chemical moiety that may be bifunctional or multifunctional, and is used to link the bispecific antibody or the antigen-binding fragment thereof of the present application to the drug of the present application. The linker may include one conjugating component or may include multiple conjugating components.

In the present application, the term "tumor" refers generally to the physiological condition in mammals that is typically characterized by unregulated cell growth. The tumor comprises one or more cancerous cells. The tumor may include solid tumors and/or non-solid tumors.

In the present application, the term "tumor microenvironment" refers generally to the environment in which a tumor exists, which is the non-cellular area within the tumor and the area directly outside the tumorous tissue but does not pertain to the intracellular compartment of the cancer cell itself. The tumor and the tumor microenvironment are closely related and can interact constantly. For example, a tumor can change the tumor microenvironment, and the tumor microenvironment can affect the growth and spread of the tumor. Typically, the tumor microenvironment has a low pH in the range of 5.8 to 7.0. The tumor microenvironment may also have a lower concentration of glucose and other nutrients, but a higher concentration of lactic acid. Furthermore, the tumor microenvironment can have a temperature that is 0.3 to 1 °C higher than the normal physiological temperature. The tumor microenvironment has been discussed in Gillies et al., "MRI of the Tumor Microenvironment", Journal of Magnetic Resonance Imaging, vol. 16, pp. 430-450, 2002.

In the present application, the term "treatment" refers generally to both therapeutic treatment and prophylactic or preventative measures, wherein the object is to prevent or slow down (lessen) the unwanted pathologic change or disorder, such as the growth, development or spread of a hyperproliferative condition such as cancer. For the purposes of the present invention, beneficial or desired clinical results include, but are not limited to, alleviation of symptoms, diminishment of the extent of the disease, stabilization (i.e., not worsening) of the state of the disease, delay or slowing of disease progression, amelioration or palliation of the state of the disease, and remission (whether partial or total), whether detectable or undetectable. "Treatment" or "treating" can also mean prolonging survival relative to expected survival if not receiving treatment. Subjects in need of treatment include subjects already afflicted with the disease or disorder, as well as subjects predisposed to the disease or disorder or subjects for whom the condition or disorder is to be prevented.

### Detailed Description of the Invention

In one aspect, the present application provides an antibody-drug conjugate, comprising a HER2-targeting bispecific antibody or an antigen-binding fragment thereof.

In certain embodiments, the antibody-drug conjugate may comprise a structure represented by formula 1: M-(L1)ₐ-(L2)_{b}-D (formula 1), wherein M represents the HER2-targeting bispecific antibody or the antigen-binding fragment thereof of the present application; L1 represents a linker linking to M, and L2 represents a linker linking to D; a and b are each independently selected from 0-10; D represents a drug.

### HER2-Targeting Bispecific Antibody or Antigen-Binding Portion Thereof

In the present application, the HER2-targeting bispecific antibody or the antigen-binding fragment thereof can specifically bind to at least one epitope of human HER2. For example, it can specifically bind to 2 different epitopes of human HER2. In the present application, the at least two epitopes may be located in the same domain of the human HER2 protein; for example, they may also be located in at least two different domains of the human HER2 protein.

For example, the HER2-targeting bispecific antibody or the antigen-binding fragment thereof can specifically bind to extracellular domain II of human HER2 and/or extracellular domain IV of human HER2. For example, the HER2-targeting bispecific antibody or the antigen-binding fragment thereof can specifically bind to extracellular domain II of human HER2 and extracellular domain IV of human HER2.

In the present application, the HER2-targeting bispecific antibody or the antigen-binding fragment thereof may comprise a first light chain and a second light chain.

In the present application, the first light chain may comprise first LCDR1-3, wherein the first LCDR1 may comprise an amino acid sequence set forth in SEQ ID NO: 4.

In the present application, the first LCDR2 may comprise an amino acid sequence set forth in SEQ ID NO: 5.

In the present application, the first LCDR3 may comprise an amino acid sequence set forth in SEQ ID NO: 6.

For example, in the first light chain, the first LCDR1 may comprise an amino acid sequence set forth in SEQ ID NO: 4; the first LCDR2 may comprise an amino acid sequence set forth in SEQ ID NO: 5; and the first LCDR3 may comprise an amino acid sequence set forth in SEQ ID NO: 6.

In the present application, the first light chain is capable of binding to the heavy chain of pertuzumab.

In the present application, the second light chain may comprise second LCDR1-3, wherein the second LCDR1 may comprise an amino acid sequence set forth in SEQ ID NO: 1.

In the present application, the second LCDR2 may comprise an amino acid sequence set forth in SEQ ID NO: 2.

In the present application, the second LCDR3 may comprise an amino acid sequence set forth in SEQ ID NO: 3.

For example, in the second light chain, the second LCDR1 may comprise an amino acid sequence set forth in SEQ ID NO: 1; the second LCDR2 may comprise an amino acid sequence set forth in SEQ ID NO: 2; and the second LCDR3 may comprise an amino acid sequence set forth in SEQ ID NO: 3.

In the present application, the second light chain is capable of binding to the heavy chain of trastuzumab.

In the present application, the first light chain and/or the second light chain may be obtained by modifying two original monoclonal antibodies (which may be, for example, known monoclonal antibodies). In the present application, the two original monoclonal antibodies can target HER2. For example, the two original monoclonal antibodies can specifically bind to at least one epitope of human HER2. For example, the two original monoclonal antibodies can each specifically bind to 2 different epitopes of human HER2. For example, the two original monoclonal antibodies can specifically bind to extracellular domain II of human HER2 and extracellular domain IV of human HER2, respectively. For example, the two original monoclonal antibodies can be trastuzumab and pertuzumab.

For example, the first light chain and/or the second light chain may differ in amino acid sequence from the light chain of either of the two original monoclonal antibodies. As another example, the first light chain and/or the second light chain may be identical to the amino acid sequence of the light chain of either of the two original monoclonal antibodies, or the amino acid sequence(s) of the first light chain and/or the second light chain may be obtained by modifying the amino acid sequence of the light chain of either of the two original monoclonal antibodies. For example, the modification may include amino acid sequence modifications. For example, the purpose of the modification may be to maintain as much affinity as possible for the antigens or antigenic epitopes corresponding to the two original monoclonal antibodies.

In the present application, the modification may include mutation, deletion or addition of amino acids, for example, mutation, deletion or addition of no more than 3 amino acids, no more than 2 amino acids, or no more than 1 amino acid.

In the present application, the amino acid sequence of a variable region of the first light chain may be identical to the amino acid sequence of a variable region of the second light chain.

In the present application, a variable region of the first light chain and the second light chain may comprise an amino acid sequence set forth in any one of SEQ ID NOs: 7-12.

In the present application, a variable region of the first light chain and the second light chain may comprise an amino acid sequence set forth in SEQ ID NO: 7.

In the present application, the first light chain and the second light chain may comprise a light chain constant region. The light chain constant region may be of type κ or λ. For example, the κ-type light chain constant region includes various allotypes, such as Km1, Km1,2, and Km3; the λ-type light chain constant region includes various allotypes, such as CL1, CL2, CL3, CL6, and CL7.

In the present application, the first light chain and the second light chain may comprise an amino acid sequence set forth in any one of SEQ ID NOs: 13-18.

In the present application, the first light chain may be selected from the group consisting of: the light chain of pertuzumab or a mutant thereof and the light chain of trastuzumab or a mutant thereof. In the present application, the second light chain may be selected from the group consisting of: the light chain of pertuzumab or a mutant thereof and the light chain of trastuzumab or a mutant thereof.

In the present application, the first light chain may be the light chain of pertuzumab or a mutant thereof.

In the present application, the second light chain may be the light chain of trastuzumab or a mutant thereof.

In the present application, the amino acid sequences of the first light chain and the second light chain may be identical.

In the present application, the first light chain and the second light chain may comprise an amino acid sequence set forth in SEQ ID NO: 13.

In the present application, the HER2-targeting bispecific antibody or the antigen-binding fragment thereof may comprise a first heavy chain and a second heavy chain, wherein the first heavy chain is capable of properly binding to the first light chain under physiological conditions or in an *in vitro* protein expression state.

In the present application, the second heavy chain is capable of properly binding to the second light chain under physiological conditions or in an *in vitro* protein expression state.

In the present application, the first heavy chain may comprise a first heavy chain variable region, and the first heavy chain variable region may be the heavy chain variable region of pertuzumab.

In the present application, the second heavy chain may comprise a second heavy chain variable region, and the second heavy chain variable region may be the heavy chain variable region of trastuzumab.

In the present application, the first heavy chain and the second heavy chain may comprise a heavy chain constant region, wherein the heavy chain constant region may be derived from the constant region of a human IgG.

In certain cases, the heavy chain constant region of the first heavy chain, and/or the heavy chain constant region of the second heavy chain, may comprise an Fc region. The Fc region may be modified; for example, the modification can increase the ratio of heterodimer formation by the first heavy chain and the second heavy chain.

In the present application, techniques for the modification may be well known to those skilled in the art; see, e.g., Ridgway, Presta et al. 1996; Carter 2001, patent CN 102558355A, and patent CN 103388013A.

In the present application, the heavy chain constant region of the first heavy chain and the heavy chain constant region of the second heavy chain may or may not be of the same heavy chain type. For example, the heavy chain constant region of the first heavy chain and the heavy chain constant region of the second heavy chain may differ in amino acid sequence from the heavy chain constant regions of the two original monoclonal antibodies. As another example, the amino acid sequence of the heavy chain constant region of the first heavy chain may be identical to the amino acid sequence of the heavy chain constant region of one of the original monoclonal antibodies; and/or the amino acid sequence of the heavy chain constant region of the second heavy chain may be identical to the amino acid sequence of the heavy chain constant region of the other original monoclonal antibody.

In the present application, an Fc-fragment of the first heavy chain and the second heavy chain may comprise an amino acid sequence set forth in any one of SEQ ID NOs: 24-57.

In the present application, the first heavy chain may comprise an amino acid sequence set forth in any one of SEQ ID NOs: 21-24. For example, the first heavy chain may comprise an amino acid sequence set forth in SEQ ID NO: 21 or 23.

In the present application, the second heavy chain may comprise an amino acid sequence set forth in any one of SEQ ID NOs: 21-24. For example, the second heavy chain may comprise an amino acid sequence set forth in SEQ ID NO: 22 or 24.

In the present application, the first heavy chain may comprise an amino acid sequence set forth in SEQ ID NO: 21 or 23, and the second heavy chain may comprise an amino acid sequence set forth in SEQ ID NO: 22 or 24. For example, in the present application, the first heavy chain may comprise an amino acid sequence set forth in SEQ ID NO: 21, and the second heavy chain may comprise an amino acid sequence set forth in SEQ ID NO: 22. For example, in the present application, the first heavy chain may comprise an amino acid sequence set forth in SEQ ID NO: 23, and the second heavy chain may comprise an amino acid sequence set forth in SEQ ID NO: 24.

In the present application, the HER2-targeting bispecific antibody or the antigen-binding fragment thereof can be obtained by conventional techniques and means in the art. For example, it can be obtained by purification from a host cell. Methods for the purification may include chromatographic techniques such as size exclusion, ion exchange, affinity chromatography, and ultrafiltration.

### Drug Conjugate and Compound

In the present application, the antibody-drug conjugate may comprise a structure represented by formula 1: M-(L1)ₐ-(L2)_{b}-D (formula 1), wherein M represents the HER2-targeting bispecific antibody or the antigen-binding fragment thereof of the present application; L1 represents a linker linking to M, and L2 represents a linker linking to D; a and b are each independently selected from 0-10; D represents a drug.

In the present application, provided is a compound for preparing the antibody-drug conjugate of the present application, which has a structure represented by formula 2: M-(L1)ₐ (formula 2), wherein M represents the HER2-targeting bispecific antibody or the antigen-binding fragment thereof of the present application; L1 represents a linker linking to M; a is selected from 0-10.

### Modification of M and Linking to L1

In the present application, the L1 and the M may be linked by a sulfhydryl, azido or amide group on the M.

In the present application, the M may comprise a first heavy chain and a second heavy chain, and the first heavy chain and/or the second heavy chain may comprise a linking site capable of linking to the L1.

In the present application, the linking site may comprise a group capable of linking to the L1 following deglycosylation modification.

In the present application, the group may be located at the side group of the amino acid Q at position 297 of the first heavy chain; and/or may be located at the side group of the amino acid Q at position 298 of the second heavy chain. For example, the group may include the amide group. For example, the amino acid Q can be used as the linking site using transglutaminases (TGs) to form the antibody-drug conjugate with a DAR of about 2.

In the present application, the linking site may comprise a group capable of linking to the L1 following glycosylation modification.

In the present application, the group may be located at the side group of the amino acid N at position 299 of the first heavy chain; and/or may be located at the side group of the amino acid N at position 300 of the second heavy chain. Within the CH2 domain of the heavy chain, N299 or N300 may have a conserved glycosylation site. The glycosylation site at the N can be used to perform site-specific conjugation.

The numbering of the positions of the amino acids may start from the amino acid at the N-terminus of the first heavy chain and/or the second heavy chain.

In the present application, on the glycosylation modification site, glycosylation modification can be performed. In the present application, the glycosylation pattern can be accomplished, for example, by expressing the protein in a cell with an altered glycosylation structure. Cells with altered glycosylation structures have been described in the art and can be used to express therein a protein with the glycosylation modification (e.g., the HER2-targeting bispecific antibody or the antigen-binding fragment thereof of the present application).

Protein glycosylation can depend on the amino acid sequence of a protein (e.g., the HER2-targeting bispecific antibody or the antigen-binding fragment thereof of the present application) and the host cell in which the protein is expressed. Different organisms may produce different glycosylation enzymes (e.g., glycosyltransferases and glycosidases), and have different substrates (nucleotide sugars) available. Due to these factors, the protein glycosylation pattern and the composition of the residue of the glycosylation modification site may vary depending on the host system in which the particular protein is expressed. Glycosyl residues useful in the present application may include glucose, galactose, mannose, fucose, n-acetylglucosamine and sialic acid. For example, the glycosylation modification may include patterns of glycosylation modifications adapted to humans.

In the present application, the glycosylation modification can effect a change in the properties of a protein (e.g., the HER2-targeting bispecific antibody or the antigen-binding fragment thereof of the present application).

Different protein glycosylations can affect and/or result in different protein properties (e.g., changes in properties such as expression level, in vivo half-life, protein folding, solubility, susceptibility to proteases, trafficking, transport, compartmentalization, secretion, recognition by other proteins or factors, antigenicity, or allergenicity).

In the present application, the specific structure and preparation method of the protein glycosylation can be adjusted according to different glycosylation purposes, and those skilled in the art can adjust based on existing techniques in the art. In certain cases, the glycosylation modification can be adjusted based on the species specificity of humans and/or animals. In certain cases, the glycosylation modification can be achieved with the help of a glycosylase (e.g., a heterologous glycosylase derived from the host cell). For example, the glycosylation modification can result in a protein that exhibits human protein glycosylation characteristics (e.g., the HER2-targeting bispecific antibody or the antigen-binding fragment thereof of the present application). The glycosylase may be of natural or non-natural origin.

In the present application, the glycosylation modification may comprise: the M having been contacted with UDP-GalNAz, β-1,4-galactosyltransferase or a variant thereof. For example, the β-1,4-galactosyltransferase or a variant thereof (e.g., β-1,4-Gal-T1-Y289) may have the ability to integrate modified N-acetylgalactosamine (GalNAc), and may be linked to a terminal N-acetylglucosamine (GlcNAc) residue on a glycan of a protein (e.g., the HER2-targeting bispecific antibody or the antigen-binding fragment thereof).

In the present application, after contacting with UDP-GalNAz, β-1,4-galactosyltransferase or a variant thereof, the M may comprise the group of the present application. For example, the side group of the amino acid N at position N299 of the first heavy chain of the M; and/or the side group of the amino acid N at position N300 of the second heavy chain of the M may obtain a -N₃ group modification, such that the M becomes glycosylation modified.

For example, in the antibody-drug conjugate of the present application and/or the compound of the present application, an Fc region of the M may be linked to the L1 by the group. For example, in the antibody-drug conjugate of the present application and/or the compound of the present application, the M may be linked to the L1 by the group. For example, the glycosylation moditied M can undergo an addition reaction with the L1.

### Linker

In the present application, the a may be an integer selected from 0-10, for example, 0, 1, 2, 3, 4, 5, 6, 7, 8, 9 or 10.

In the present application, the b may be an integer selected from 0-10, for example, 0, 1, 2, 3, 4, 5, 6, 7, 8, 9 or 10.

In the present application, the L1 and/or L2 may be selected from the group consisting of: a cleavable linker, a non-cleavable linker, a hydrophilic linker, a hydrophobic linker, a charged linker, an uncharged linker, and a dicarboxylic acid-based linker.

In the present application, both the L1 and the L2 may be considered to belong to linkers.

In the present application, the linker may be a moiety that extends the drug linkage to avoid, for example, shielding the active site of the antibody or improving the solubility of the ADC. The linker may comprise a stretcher unit and/or an amino acid unit.

In the present application, the linker may be used to conjugate (e.g., covalently link) an antibody (e.g., the HER2-targeting bispecific antibody or the antigen-binding fragment thereof of the present application) to the drug.

For example, the conjugation may be achieved by a cysteine sulfhydryl or an amine (e.g., N-terminus or amino acid side chain such as lysine) of the antibody forming a bond with a functional group of the linker.

For example, the linker may have a functional group capable of reacting with a free cysteine present in the antibody to form a bond (e.g., a covalent bond). For example, the functional group may include: maleimide, haloacetamides, α-haloacetyl, activated esters such as succinimide esters, 4-nitrophenyl esters, pentafluorophenyl esters, tetrafluorophenyl esters, anhydrides, acid chlorides, sulfonyl chlorides, isocyanates and/or isothiocyanates.

In some cases, the linker may include functional groups capable of reacting with an electrophilic group present in the antibody. For example, the functional group may include: aldehyde, ketone carbonyl, hydrazine, oxime, amino, hydrazine, thiosemicarbazone, hydrazine carboxylate and/or arylhydrazide.

In the present application, the linker may include cleavable linkers and non-cleavable linkers. For example, the linker (e.g., L2) may be a cleavable (e.g., self immolative) linker, which may facilitate the release of the drug. For example, the linker (e.g., L1) may be a non-cleavable linker, which may link to the antibody and is non-cleavable. In the present application, the cleavable linker may be cleaved under intracellular conditions. For example, the cleavable linker may include: acid-labile linkers (e.g., comprising hydrazone), protease-sensitive linkers (e.g., peptidase-sensitive), photolabile linkers and/or disulfide-containing linkers. For example, the cleavable linker may include peptide linkers capable of being cleaved by an intracellular protease (e.g., a lysosomal protease) and/or an endosomal protease. In certain cases, the linker is cleavable under intracellular conditions. For example, cleavage of the linker may allow the drug in the antibody-drug conjugate to exert an effective therapeutic effect.

In the present application, the structure and/or properties of the linker are stable outside cells. The antibody-drug conjugate of the present application may be structurally stable before transport or delivery into a cell. For example, in the antibody-drug conjugate, the HER2-targeting bispecific antibody or the antigen-binding fragment thereof remains conjugated to the drug.

In certain cases, if the linker in the antibody-drug conjugate is capable of, e.g., maintaining the specific binding properties of the HER2-targeting bispecific antibody or the antigen-binding fragment thereof; taking part in the delivery of the antibody-drug conjugate; and/or maintaining the therapeutic effect (e.g., cytotoxic effect) of the drug.

In the present application, the linker may include hydrophilic linkers (e.g., PEG4Mal and sulfo-SPDB) and hydrophobic linkers. For example, the hydrophilic linker can reduce the extent to which the antibody-drug conjugate may be pumped out of resistant cancer cells through MDR (multiple drug resistance) or functionally similar transporters.

In the present application, the linker can also function to inhibit cell growth and/or cell proliferation, either directly or indirectly. For example, the linker can function as an intercalator in the cleavage. For example, the linker can act indirectly to inhibit macromolecular biosynthesis.

In the present application, the linker can facilitate entry of the antibody-drug conjugate into a cell (e.g., can facilitate an "internalization" effect). In the present application, the linker can also be designed to improve the stability of the antibody-drug conjugate.

In the present application, the L1 is capable of taking part in a SPAAC reaction. The SPAAC reaction is an azide-alkynyl cycloaddition reaction. The SPAAC reaction can be used as one of the addition reactions and applied to the preparation of the antibody-drug conjugate.

In the present application, the L1 may be selected from the group consisting of: maleimide, succinimide-3-yl-N, and DBCO.

In the present application, the L1 may be DBCO-(PEG)ₙ₁, wherein ₙ₁ is an integer from 0-10, or the L1 is maleimide. For example, the L1 may be DBCO-(PEG)₄ or DBCO-(PEG)₃. In the present application, the PEG molecule can be used to increase the hydrophilicity of the antibody-drug conjugate. The PEG molecule can be used to meet the spatial distance requirements for L2 (e.g., GGFG) cleavage (e.g., enzymatic cleavage).

In the present application, the L2 may be selected from the group consisting of: a polypeptide, VC-PAB, N-succinimidyl-3-(2-pyridyldithio)propionate (SPDP), N-succinimidyl 4-(2-pyridyldithio)valerate (SPP), N-succinimidyl 4-(2-pyridyldithio)butyrate (SPDB), N-succinimidyl-4-(2-pyridyldithio)-2-sulfobutyrate (sulfo-SPDB), N-succinimidyl iodoacetate (SIA), N-succinimidyl (4-iodoacetyl)aminobenzoate (SIAB), maleimide PEG NHS, N-succinimidyl 4-(maleimidomethyl)cyclohexylcarboxylate (SMCC), sulfosuccinimidyl 4-(N-maleimidomethyl)cyclohexane-1-carboxylate (sulfo-SMCC) and 2,5-dioxopyrrolidin-1-yl 17-(2,5-dioxo-2,5-dihydro-1H-pyrrol-1-yl)-5,8,11,14-tetraoxy-4,7,10,13-tetraazaoctadecan-1-o ate (CX1-1).

In the present application, the L2 may be GGFG, LP, VK, VC, GFG, GGFGG, GGFGS, GGFGGG, GGFGGE, GGFGGGFG, DGGF, DGGFG, D^{d} GGFG, DG ^{Me} GFG, DGGFS, DDGGFG, KDGGFG, KGGFG, EGGFG or SGGFG. For example, the L2 may be GGFG. The cathepsin B-cleavable tetrapeptide Gly-Gly-Phe-Gly (GGFG) is a highly hydrophilic linker (e.g., more hydrophilic than Gly-Phe-Leu-Gly).

### Drug

In the present application, the drug may have an ability to kill tumor cells, and/or an ability to inhibit tumor cell growth.

In the present application, the drug may include small-molecule drugs.

In the present application, the drug may be selected from the group consisting of: a V-ATPase inhibitor, a Bcl2 inhibitor, an MCL1 inhibitor, an HSP90 inhibitor, an IAP inhibitor, an mTor inhibitor, a microtubule stabilizer, a microtubule destabilizer, auristatin, dolastatin, a maytansinoid, MetAP (methionine aminopeptidase), an inhibitor of nuclear export of protein CRM1, a DPPIV inhibitor, a proteasome inhibitor, an inhibitor of phosphoryl transfer reactions in mitochondria, a protein synthesis inhibitor, a CDK2 inhibitor, a CDK9 inhibitor, a kinesin inhibitor, an HDAC inhibitor, a DNA damaging agent, a DNA alkylating agent, a DNA intercalator, a DNA minor groove binder, a DHFR inhibitor, a nucleoside analog, an HD AC inhibitor, an anthracycline, a NAMPT inhibitor, SN-38 glucuronic acid, etoposide phosphate, nitrogen mustard, a proteosome inhibitor, a cytokine, and a Toll-like receptor agonist.

In the present application, the drug may be selected from the group consisting of: DM1, exatecan, DXd, MMAE, SN-38, calicheamicin, anthracyclin-5G, DM4, microtubule inhibitor SHR153024, PNU-159682, Duo5 toxin, an SN38 derivative or derivatives thereof.

In the present application, the drug may be camptothecin or a derivative thereof. In the present application, the drug may be a DNA topoisomerase I inhibitor.

In the present application, the drug may be exatecan (DX-8951), DXd, duocarmycin, mitomycin C, tallysomycin, maytansine, a TLR7 agonist, a TLR8 agonist, a TLR7/8 agonist, or a TLR9 agonist.

For example, the drug may have the following structure:

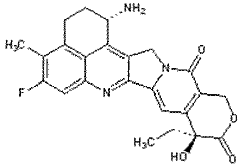

In the present application, the antibody-drug conjugate may have a structure selected from the group consisting of: 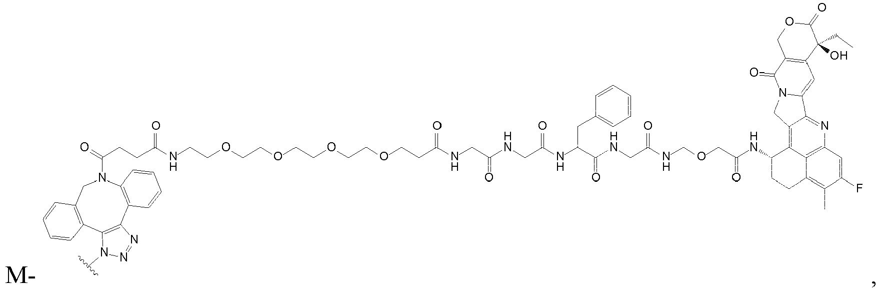 and 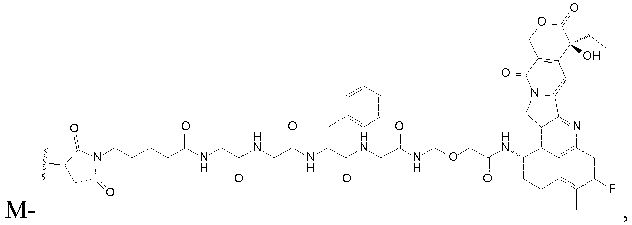 and

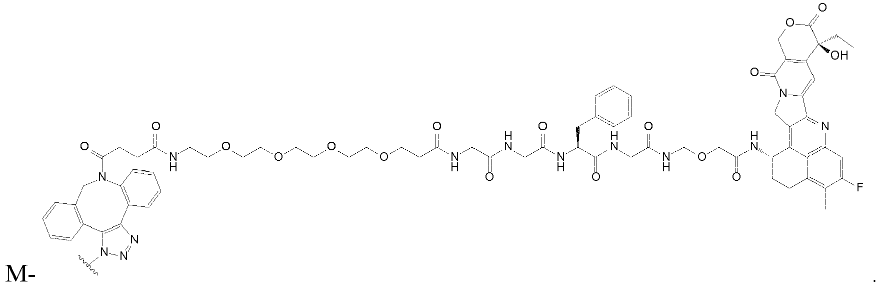

In the present application, the drug/antibody ratio of the antibody-drug conjugate may be about 2-6. For example, the drug/antibody ratio may be about 1, about 2, about 3, about 4, about 5, or about 6. For example, the drug/antibody ratio may be 4. For example, the drug/antibody ratio may be 3.

### Compound and Preparation Method

In another aspect, the present application provides a compound for the antibody-drug conjugate of the present application, which has a structure represented by formula 2: M-(L1)ₐ (formula 2), wherein M represents the HER2-targeting bispecific antibody or the antigen-binding fragment thereof of the present application; L1 represents a linker linking to M; a is selected from 0-10.

In the present application, the compound may comprise a structure selected from the group consisting of:

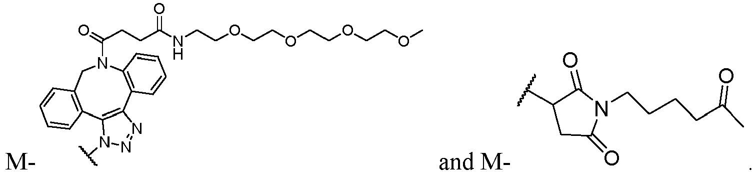

In another aspect, the present application provides a method for preparing the antibody-drug conjugate of the present application, comprising the following step: contacting the compound of the present application with the drug of the present application.

In some cases, the method for preparing the antibody-drug conjugate may comprise the following steps: (1) contacting the L1 and the L2 of the present application with the drug; (2) obtaining a compound (L1)ₐ-(L2)_{b}-D; and (3) contacting the M of the present application with the compound (L1)ₐ-(L2)_{b}-D to obtain the antibody-drug conjugate of the present application. For example, the M in step (3) is subjected to the glycosylation modification.

### Pharmaceutical Composition and Use

In another aspect, the present application provides a pharmaceutical composition, comprising the antibody-drug conjugate of the present application, or a pharmaceutically acceptable carrier. In the present application, the pharmaceutical composition may comprise a "therapeutically effective dose" or a "prophylactically effective dose" of the antibody-drug conjugate of the present application. The "therapeutically effective dose" refers to an amount effective, at dosages and for periods of time necessary, to achieve the desired therapeutic result. The therapeutically effective dose can be determined by those skilled in the art and, for example, may vary depending on such factors as the state of the disease, the age, sex, and body weight of the subject, and the ability of the antibody-drug conjugate to elicit a desired response in the subject. The "prophylactically effective dose" refers to an amount effective, at dosages and for periods of time necessary, to achieve the desired prophylactic result. For example, the prophylactically effective dose may be lower than the therapeutically effective dose.

In the present application, the pharmaceutical composition may be formulated in a form suitable for administration (e.g., suitable for parenteral, intradermal, intramuscular, intraperitoneal, intravenous and subcutaneous, intratumoral and/or mucosal administration). In the present application, the pharmaceutical composition may comprise other pharmaceutically active ingredients.

In the present application, the pharmaceutically acceptable carrier may include any and all solvents, dispersion media, coatings, antibacterial and antifungal agents, isotonic agents and absorption delaying agents, and the like, that are physiologically compatible. The pharmaceutically acceptable carrier may include one or more of water, saline, phosphate-buffered saline, dextrose, glycerol, ethanol, and the like, as well as combinations thereof. The pharmaceutically acceptable carrier may also include isotonic agents, wetting agents, emulsifying agents, preservatives and/or buffering agents.

In another aspect, the present application provides a method for modulating a tumor microenvironment in a subject, comprising the following step: administering to the subject the antibody-drug conjugate of the present application, or the pharmaceutical composition of the present application.

In another aspect, the present application provides a method for modulating an immune response in a subject, comprising the following step: administering to the subject the antibody-drug conjugate of the present application, or the pharmaceutical composition of the present application.

The present application provides use of the antibody-drug conjugate of the present application or the pharmaceutical composition of the present application in the preparation of a medicament, wherein the medicament can modulate a tumor microenvironment in a subject and/or modulate an immune response in a subject.

The present application provides the antibody-drug conjugate, or the pharmaceutical composition of the present application for use in modulating a tumor microenvironment in a subject and/or modulating an immune response in a subject.

In another aspect, the present application provides use of the antibody-drug conjugate of the present application or the pharmaceutical composition of the present application in the preparation of a medicament, wherein the medicament can prevent and/or treat tumors.

The present application provides a method for preventing and/or treating tumors, comprising the following step: administering to a subject a medicament prepared with the antibody-drug conjugate of the present application or the pharmaceutical composition of the present application.

The present application provides the antibody-drug conjugate, or the pharmaceutical composition of the present application for use in preventing and/or treating tumors.

In the present application, the tumors may include solid tumors and/or non-solid tumors.

For example, the tumors may include gastric cancer, breast cancer (e.g., ductal breast cancer) and/or pancreatic cancer. For example, the tumors of the present application may include HER2 weakly positive tumors and/or HER2 negative tumors; for example, the technical solutions of the present application may have a bystander killing effect.

Without being bound by any theory, the following examples are intended only to illustrate various technical solutions of the invention in the present application, but not to limit the scope of the present application.

### Examples

A HER2-targeting bispecific antibody (hereinafter referred to as antibody A), wherein antibody A comprises one first light chain, one second light chain, one first heavy chain and one second heavy chain, wherein the amino acid sequences of the first light chain and the second light chain are set forth in SEQ ID NO: 13, and wherein the amino acid sequence of the heavy chain variable region of the first heavy chain is set forth in SEQ ID NO: 19; the amino acid sequence of the heavy chain variable region of the second heavy chain is set forth in SEQ ID NO: 20. The amino acid sequence of the first heavy chain is set forth in SEQ ID NO: 21; the amino acid sequence of the second heavy chain is set forth in SEQ ID NO: 22.

According to the amino acid sequences of antibody A, antibody A was artificially synthesized, separated and purified.

DS-8201 (also known as T-DXd, trade name ENHERTU) was prepared by Daiichi Sankyo and AstraZeneca. DS-8201 was formed by conjugating an anti-HER2 IgG1 mAb with the topoisomerase I inhibitor DXd by a connector.

U3-1402 was prepared by Daiichi Sankyo. U3-1402 was formed by conjugating the HER3 antibody patritumab with the topoisomerase inhibitor DXd by a covalent bond.

### Example 1. Preparation of Antibody A-ADC1 Molecule

### (1) Preparation of a compound comprising a linker and a toxin

DXd (i.e., an exatecan DX-8951 derivative, purchased from Levena Biopharma) and compound 1, DBCO-PEG4-GGFG-DXd, which comprises a linker and a toxin, were obtained. Compound 1 has the following structure formula:

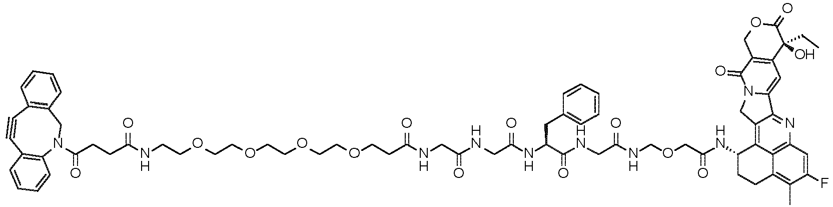

### (2) Preparation of glycosylated antibody A

Antibody A was contacted with β-1,4-galactosyltransferase (β-1,4-al-T1) (purchased from Shanghai BioChemSyn) in the presence of UDP-GalNAz. The structural formula of UDP-GalNAz is

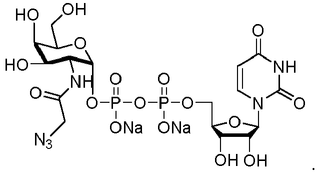

After contact, antibody A obtained -N₃ group modifications at position N299 of the first heavy chain and position N300 of the second heavy chain, becoming a glycosylation modified antibody A. The reaction for the glycosylation modification of antibody A is shown in FIG. 1. The resulting glycosylation modified antibody A was analyzed by HPLC, and the analysis results are shown in FIG. 2. The results in FIG. 2 show that the glycosylation modified antibody A has a unique characteristic peak and the molecular weight is 148,796 Da.

### (3) Preparation of antibody A-ADC1 molecule

The DBCO-PEG4-GGFG-DXd in step (1) was contacted and reacted with the glycosylation modified antibody A prepared in step (2), at a temperature of 25-35 °C for 10-15 h, wherein DBCO underwent SPAAC reactions with the -N₃ groups in the glycosylation modified antibody A, affording an antibody A-ADC1 molecule.

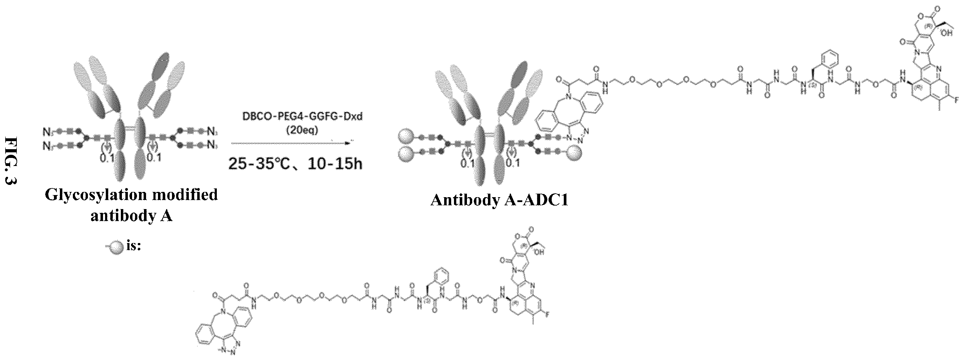

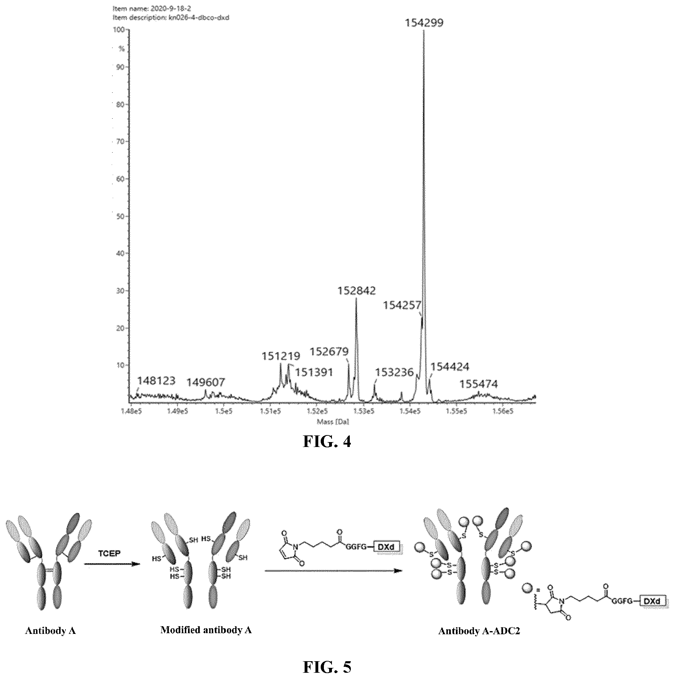

The reaction for obtaining the antibody A-ADC1 molecule is shown in FIG. 3. Antibody A-ADC1 was analyzed by HPLC, and the analysis results are shown in FIG. 4. The results in FIG. 4 show that the antibody A-ADC1 molecule has a unique characteristic peak and the molecular weight is 154,299.

### (4) Preparation of herceptin-ADC1 molecule

Herceptin was subjected to glycosylation modification according to the method described in Example 1 and reacted with DBCO-PEG4-GGFG-DXd to obtain a herceptin-ADC1 molecule with a DAR of 8.

### Example 2. Preparation of Antibody A-ADC2 Molecule

### (1) Preparation of a compound comprising a linker and a toxin

Maleimide was reacted with GGFG-DXd to obtain compound 2, which comprises a linker and a toxin. The structural formula of compound 2 is:

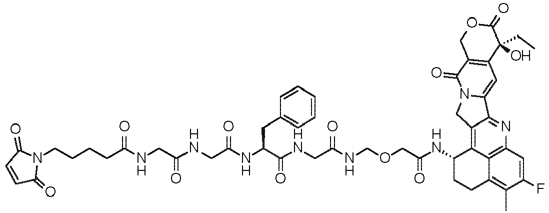

### (2) Preparation of modified antibody A

A modified antibody A was obtained by introducing sulfhydryl groups (e.g., 8 -SH groups) into antibody A through a reaction of antibody A with TECP.

### (3) Preparation of antibody A-ADC2 molecule

The modified antibody A prepared in step (2) was reacted with the compound 2 prepared in step (1), which comprises a linker and a toxin, to obtain an antibody A-ADC2 molecule.

The reaction for obtaining the antibody A-ADC2 molecule is shown in FIG. 5.

### Example 3. Analysis of Stability of Antibody A-Containing ADC Molecules

The stability of the antibody A-ADC1 prepared in Example 1, the antibody A-ADC2 prepared in Example 2, and DS-8201 in human serum was investigated.

### (1) Stability of antibody A-ADC1 in serum

Antibody A-ADC1 was purified from serum using Protein L, and after 3 days and 7 days, no significant thiol exchange was observed.

The specific steps of the experiment: 100-µL samples of treated human serum were added to 1.5-mL centrifuge tubes, and antibody A-ADC1 was added to achieve a final concentration of 0.1 mg/mL. The tubes were incubated in a 37 °C incubator for 3 days and 7 days, respectively. 20 µL of Protein L magnetic bead suspension was added to a 1.5-mL centrifuge tube, and the tube was placed on a magnetic rack to separate and remove the preservation solution. 200 µL of DPBS was added and well mixed with the magnetic beads, and the tube was placed on a magnetic rack to separate and remove the supernatant (this step was repeated twice).

The antibody A-ADC1 which had been incubated for 3 days or 7 days was added to the 1.5-mL centrifuge tube containing magnetic beads and well mixed. The tube was left in a mixer at room temperature for 1 h and then placed on a magnetic rack to remove the supernatant. Next, 200 µL of DPBS was added and well mixed, and the tube was placed on a magnetic rack to separate and remove the supernatant (this step was repeated 3 times). Finally, 20 µL of eluent (100 mM glycine, pH = 2.8) was added and well mixed at room temperature on the magnetic rack. The supernatant was collected, and 20 µL of neutralization buffer (200 mM tris, pH = 8.0) was added to the collected supernatant. The concentration was measured, and the purified antibody A-ADC1 was analyzed on a Waters Xevo G2-QTOF mass spectrometer.

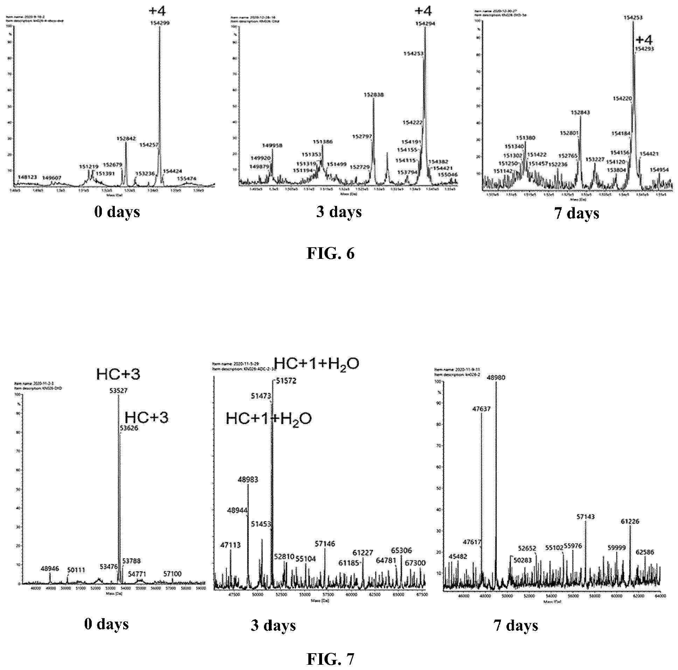

The results are shown in FIG. 6. The results in FIG. 6 show that the molecular weight of the antibody A-ADC1 (around 154,299) did not change substantially after incubation in human serum for 0 days, 3 days or 7 days: the DAR value remained at 4 after 3 days or 7 days in serum, and no significant thiol exchange occurred after the conjugation drug molecule was incubated in serum for 3 days or 7 days, indicating that antibody A-ADC1 has good stability in serum, which makes it less likely to release the toxin into the blood during *in vivo* delivery and is more conducive to delivering the conjugated small-molecule toxin drug to the tumor target, so that the risk of toxic side effects on normal body tissues caused by off-target toxin molecules can be reduced.

### (2) Stability of antibody A-ADC2 in serum

The stability of antibody A-ADC2 in serum was tested according to the method in step (1).

The results of Waters Xevo G2-QTOF mass spectrometry analysis are shown in FIG. 7. The results in FIG. 7 show that when antibody A-ADC2 was incubated in human serum for 0 days, 3 days or 7 days, after 3 days of incubation, the maleimide on the light chain stably existed in human serum due to ring-opening hydrolysis; however, after 3 days of incubation, the DAR value of the heavy chain decreased from 6 to around 2, and after 7 days of incubation, the DAR value of the heavy chain was almost 0, indicating that there was an elimination of the heavy chain of antibody A-ADC2 in serum after 7 days.

### (3) Stability of DS-8201 in serum

The stability of DS-8201 in serum was tested according to the method in step (1).

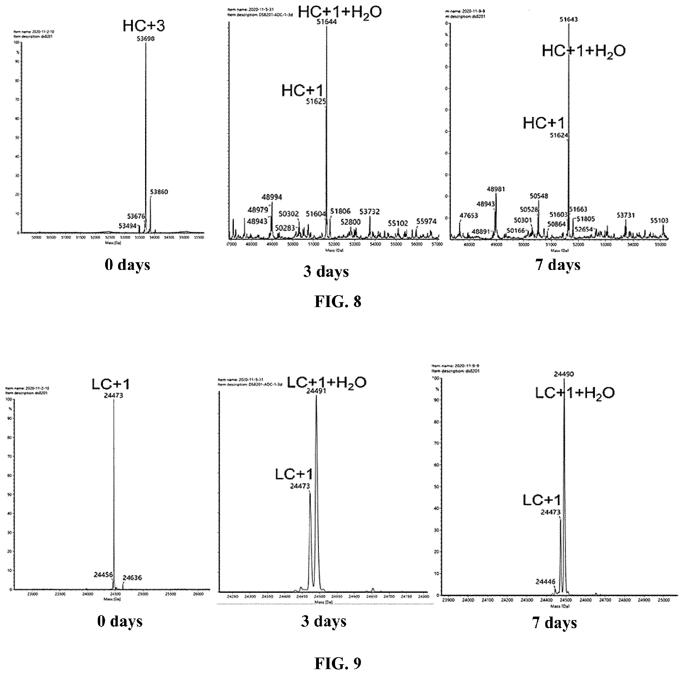

The results of Waters Xevo G2-QTOF mass spectrometry analysis of the incubation of the heavy and light chains of DS-8201 in serum are shown in FIGs. 8 and 9, respectively. The results in FIGs. 8 and 9 show that when DS-8201 was incubated in human serum for 0 days, 3 days or 7 days, after 3 days of incubation, the maleimide on the light chain stably existed in human serum due to ring-opening hydrolysis; however, after 7 days, the DAR value of the heavy chain decreased from 6 to around 2, indicating that there was a significant elimination of the heavy chain of DS-8201 in the serum, and the off-target effect of the small-molecule toxin on the heavy chain was significant.

### Example 3. Testing of Binding Affinities of Antibody A-Containing ADC Molecules for FcyR

The affinities of antibody A and the antibody A-ADC1 prepared in Example 1 for FcyRI, FcyRIIIa, etc. were determined by bio-layer interferometry (BLI) (using a Fortebio macromolecular interaction instrument from Danaher).

### The specific steps of the affinity testing:

The antibody A-WS-161018 (parent molecule antibody A) or antibody A-ADC1 was immobilized to a FAB2G biosensor at a concentration of 10 µg/mL and a height of 2 nm.

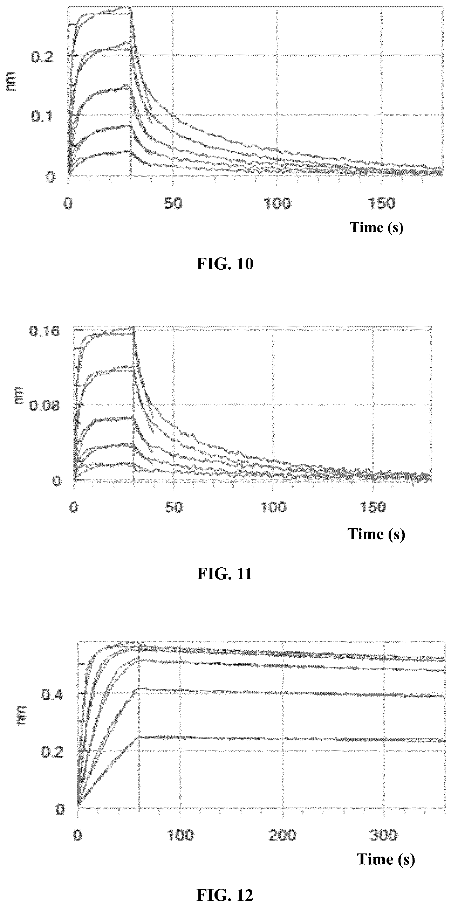

FcyRIIIa was diluted to 1000 nM, 500 nM, 250 nM, 125 nM and 62.5 nM. Observations were performed at baseline for 60 s, binding for 30 s, and dissociation for 150 s. The diluent was a kinetic buffer, the regeneration solution was glycine-HCl (pH 1.7), and the neutralization solution was a diluent. Fitting was performed with a dissociation time of 10 s. The experimental results are shown in FIGs. 10-11 and Table 1. FIGs. 10 and 11 show the binding affinities of antibody A and antibody A-ADC1 for FcyRIIIa, respectively.

**Table 1**

| Sample | KD (M) | Kon (1/Ms) | Kdis (1/s) |
|---|---|---|---|
| Antibody A | 1.65E-07 | 5.60E+05 | 9.21E-02 |
| Antibody A-ADC1 | 1.50E-07 | 5.76E+05 | 8.61E-02 |

The antibody A-WS-161018 (parent molecule antibody A) or antibody A-ADC1 was immobilized to a FAB2G biosensor at a concentration of 10 µg/mL and a height of 2 nm.

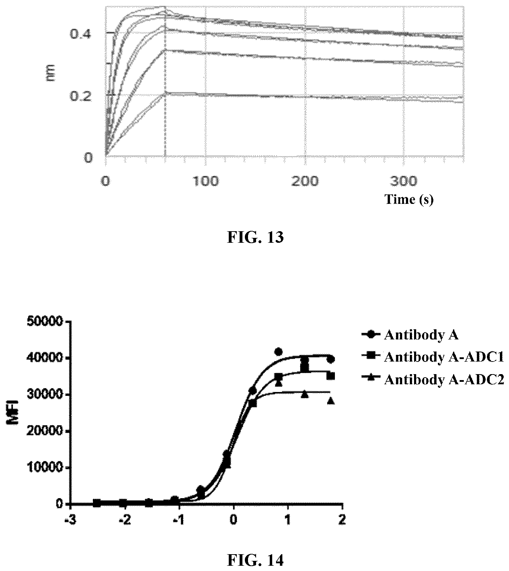

FcyRI was diluted to 200 nM, 100 nM, 250 nM, 50 nM and 25 nM. Observations were performed at baseline for 60 s, binding for 30 s, and dissociation for 150 s. The diluent was 0.02% PBST20, the regeneration solution was glycine-HCl (pH 1.7), and the neutralization solution was a diluent. Fitting was performed with a dissociation time of 10 s. The experimental results are shown in FIGs. 12-13 and Table 2. FIGs. 12 and 13 show the binding affinities of antibody A and antibody A-ADC1 for FcyRI, respectively.

**Table 2**

| Sample | KD (M) | Kon (1/Ms) | Kdis (1/s) |
|---|---|---|---|
| Antibody A | 2.89E-10 | 8.54E+05 | 2.47E-04 |
| Antibody A-ADC1 | 6.05E-10 | 9.23E+05 | 5.58E-04 |

The above results show that the affinity of antibody A-ADC1 for FcyRIIIa is consistent with that of the parent molecule antibody A; antibody A-ADC1 has a strong binding affinity for FcyRI, with a KD value of 6.05 × 10⁻¹⁰ M, and the affinity is similar to that of the parent molecule antibody A.

The FcyR binding experiment shows that the site-specific conjugation to which antibody A-ADC1 pertains does not affect the Fc function of the antibody moiety in the ADC; antibody A-ADC1 still retains the Fc function of the parent molecule antibody A; the antibody moiety in antibody A-ADC1 can work synergistically with the toxin to kill tumor cells.

### Example 4. Abilities of Antibody A-Containing ADC Molecules to Bind to Tumor Cells

The binding activities of the antibody A-ADC1 prepared in Example 1, the antibody A-ADC2 prepared in Example 2, and antibody A for NCI-N87 cells were investigated.

### The specific steps of the assay:

(1) Drugs were prepared with 1% BSA/PBS. The highest concentration prepared was 120 µg/mL (the working concentration was 60 µg/mL) and was 3-fold diluted to obtain a total of 10 concentrations.
(2) NCI-N87 cells (purchased from Chinese Academy of Sciences, Shanghai) were collected, centrifuged, counted, blocked with 5% BSA/PBS for 15 min, and added to 1.5-mL centrifuge tubes at 2 × 10⁵ cells/50 µL/tube. 50 µL of antibody drug was added to each of the tubes, and the tubes were left at 4 °C for 1 h.
(3) Cells were washed twice by centrifugation at 2000 rpm for 5 min. In each wash, 1000 µL of 1% BSA/PBS was added. After the last wash, the liquid was pipetted off, and 90 µL of APC-IgG diluted in 1%BSA/PBS at 1:100 was added to each well. The tubes were left in the dark at 4 °C for 30 min.
(4) Cells were washed twice by centrifugation at 2000 rpm for 5 min. In each wash, 1000 µL of 1% BSA/PBS was added. After the last wash, the liquid was pipetted off; 300 µL of 1% BSA/PBS was added to each of the tubes, and FACS assays were performed in the dark.

The results are shown in FIG. 14 and Table 3.

**Table 3**

| | Antibody A | Antibody A-ADC1 | Antibody A-ADC2 |
|---|---|---|---|
| Valley value | 675.3 | 485.8 | 660.8 |
| Peak value | 40787 | 36596 | 30780 |
| EC50 | 1.103 | 1.154 | 0.9369 |

The results in FIG. 14 and Table 3 show that the binding activities of antibody A at high concentrations are the highest, the binding activity of antibody A-ADC1 is slightly lower than that of antibody A, and the mean fluorescence intensities (MFI) of both of them tend to stabilize after reaching a peak; the activity of antibody A-ADC2 is relatively low. This indicates that the bindings of antibody A-ADC1 and its parent molecule antibody A to NCI-N87 cells were more stable.

From the EC50 values, it can also be seen that the binding activity of antibody A-ADC1 for NCI-N87 cells highly expressing HER2 is consistent with that of its parent molecule antibody A, with the EC50 values being around 1.1 µg/mL, indicating that antibody A-ADC1 and its parent molecule antibody A have similar binding activities for NCI-N87 cells highly expressing HER2; the peak value of antibody A-ADC1 is greater than that of antibody A-ADC2, indicating that the binding activity of antibody A-ADC1 for NCI-N87 cells highly expressing HER2 is slightly higher than that of antibody A-ADC2.

### Example 5. Abilities of Antibody A-Containing ADC Molecules to Kill Tumor Cells

The abilities of the antibody A-ADC1 prepared in Example 1, the antibody A-ADC2 prepared in Example 2, antibody A and DS-8201 to kill NCI-N87 cells and BT474 cells were investigated.

### The specific steps of the assay:

(a) NCI-N87 cells for 5-day treatment
   (1) Preparation of growth medium: RPMI-1640 + 10% FBS + 1% P/S; NCI-N87 cells were trypsinized, inoculated onto a 96-well plate at 10,000 cells/well/150 µL, and cultured overnight at 37 °C.
   (2) Preparation of experimental medium: RPMI-1640 + 0.1% FBS + 1% P/S; antibody A, antibody A-ADC1, antibody A-ADC2 and DS-8201 were formulated into solutions with the highest working concentration of 15 µg/mL, and the solutions were 3-fold diluted to obtain a total of 9 concentration points.
   (3) Drug dilutions with various concentrations were added at 150 µL/well, negative control wells were set up, and the plate was incubated at 37 °C for 72 ± 3 h.
   (4) 70 µL of supernatant was pipetted off from each well and discarded. 70 µL of newly prepared 15 µg/mL antibody A, antibody A-ADC1, antibody A-ADC2 or DS-8201 was added, and the plate was incubated at 37 °C for another 48 ± 2 h.
   (5) CCK-8 was added to the 96-well plate at 15 µL/well. The plate was gently tapped to mix the contents well and was left at 37 °C for around 2 h.
   (6) With 650 nm as a reference wavelength, the absorbance values at 450 nm were recorded.

(a) NCI-N87 and BT474 cells for 3-day treatment
   (1) Preparation of growth medium: RPMI-1640 + 10% FBS + 1% P/S; NCI-N87 or BT474 cells were trypsinized, inoculated onto a 96-well plate at 10,000 cells/well/150 µL, and cultured overnight at 37 °C.
   (2) Preparation of experimental medium: RPMI-1640 + 0.1% FBS + 1% P/S; antibody A, antibody A-ADC1, antibody A-ADC2 and DS-8201 were formulated into solutions with the highest working concentration of 30 µg/mL, and the solutions were 8-fold diluted to obtain a total of 5 concentration points.
   (3) Drug dilutions with various concentrations were added at 150 µL/well, negative control wells were set up, and the plate was incubated at 37 °C for 72 ± 3 h.
   (4) CCK-8 was added to the 96-well plate at 15 µL/well. The plate was gently tapped to mix the contents well and left at 37 °C for around 2 h.
   (6) With 650 nm as a reference wavelength, the absorbance values at 450 nm were recorded.

### The results of the assays:

### (1) NCI-N87 tumor cell killing assays

The results of the assays in which NCI-N87 cells were treated for 3 days and 5 days are shown in FIG. 15 and FIG. 16, respectively.

The results in FIGs. 15-16 show that after NCI-N87 cells were treated for 3 days according to the steps of the method described above, the inhibition rate of antibody A-ADC1 against NCI-N87 reached over 80%, the inhibition rate of antibody A came next, and the inhibition rate of DS8201 (herceptin-ADC1) reached only 60%, which is the lowest. After NCI-N87 cells were treated for 5 days, the inhibition rate of antibody A-ADC1 remained the highest, and was slightly higher than those of its parent molecule and DS-8201 (herceptin-ADC1). Thus, it can be seen that the killing effect of antibody A-ADC1 on NCI-N87 cells is significantly higher than that of DS-8201 (herceptin-ADC1).

### (2) BT474 tumor cell killing assay

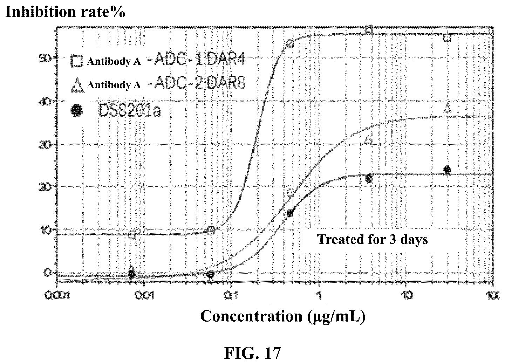

The results of the assay in which BT474 cells were treated for 3 days are shown in FIG. 17.

The results in FIG. 17 show that after BT474 cells were treated for 3 days according to the steps of the method described above, the inhibition rate of antibody A-ADC1 against BT474 cells reached 55%, the inhibition rate of antibody A-ADC2 reached 36%, and the inhibition rate of DS-8201 reached only 23%: the inhibition rate of antibody A-ADC1 against BT474 cells is far higher than those of A-ADC2 and DS-8201, and its killing effect is 2.4 times that of DS-8201.

The killing assays against NCI-N87 and BT474 tumor cells show that both antibody A-ADC1 and antibody A-ADC2 have relatively good abilities to kill high HER2 expression positive tumor cells (NCI-N87 and BT474); and the abilities of antibody A-ADC1 and antibody A-ADC2 to kill high HER2 expression positive tumor cells (NCI-N87 and BT474 cells) are better than that of DS-8201.

### Example 6. Bystander Killing Effects of Antibody A-Containing ADC Molecules on HER2 Negative Cells

ADC drugs usually first bind to antigens on the cell membranes, and then the antibody-antigen complexes enter the cells through endocytosis and form endosomes. The endosomes then further mature and merge into lysosomes. Within the lysosomes, the cytotoxic drugs are released (the linkers are decomposed by corresponding specific proteases, such as cathepsin B, or the entire ADC drugs are decomposed in the lysosomes). The resulting cytotoxic drugs penetrate the lysosome membranes and bind to DNA or microtubules, causing cell apoptosis. These drugs can also be pumped out into tumor microenvironments through transport proteins on the cell membranes and enter neighboring tumor cells, producing killing effects on them. This can lead to "bystander effects".

Antibody A-ADC1, a sugar site-specific conjugation drug of a HER2-targeting bi-epitopic antibody, uses a cleavable linker. To investigate the bystander effect of antibody A-ADC1, we used antibody A-ADC1 and DS-8201 to treat an SK-B-R-3 (high HER2 expression) single cell system, an MDA-MB-468 (no HER2 expression) single cell system, and an SK-BR-3 + MDA-MB-468 (1: 1) mixed cell system. After 3 days of treatment, the cells in all sample wells were counted, the mixed cell system was stained with the APC anti-CD340 Antibody fluorescent antibody, and flow cytometry assays were performed.

### Experimental method

In T75 culture flasks, SK-BR-3 and MDA-MB-468 cells were in the logarithmic growth phase. The media were discarded. After two washes with PBS, the liquid was pipetted off; 2 mL of trypsin was added for digestion, and the flasks were left in a 37 °C incubator for 4 min. The cell digests were transferred to 15-mL centrifuge tubes, and 8 mL of growth medium was added to stop the digestion. The cells were pipetted until single-cell suspensions were formed, and the suspensions were centrifuged at 1000 rpm for 5 min. The supernatants were discarded, and 3 mL of medium (RPMI-1640 + 1% FBS) was added to resuspend the cells. 20 µL of cell suspension was taken and mixed with 20 µL of 0.2% trypan blue.

Count: Mono-culture: The densities of the two types of cells were adjusted to 1 × 10⁵ cells/mL using a medium (RPMI-1640 + 1% FBS); each of the two types of cells was plated onto a 96-well plate at 100 µL/well, with each well containing 1 × 10⁴ cells; the plates were incubated overnight in a 37 °C, 5% CO₂ incubator. Co-culture: The densities of the two types of cells were adjusted to 2 × 10⁵ cells/mL using a medium (RPMI-1640 + 1% FBS); to each well were added 50 µL of SK-BR-3 cells and 50 µL of MDA-MB-468 cells; each well contained a total of 2 × 10⁴ cells; the plate was incubated overnight in a 37 °C, 5% CO₂ incubator.

Antibody drug preparation: The test samples were 5-fold diluted with an RPMI-1640 medium containing 1% FBS from the highest working concentration of 50 nM to obtain a total of 4 concentrations. A control group was set up at the same time, i.e., a drug-free group (negative control). The diluted test samples were added at 100 µL/well, and 100 µL of RPMI-1640 + 1% FBS medium was directly added to the negative control. The plate was incubated at 37 °C with 5% CO₂ for 72 h. The supernatants in the 96-well plate were pipetted off. After one wash with 200 µL of 1 × PBS, 20 µL of trypsin was added to each well for digestion, and the plate was left in a 37 °C incubator for 4 min. 100 µL of medium (RPMI-1640 + 10% FBS) was added to stop the digestion. The cells were pipetted until single-cell suspensions were formed, and the plate was centrifuged at 1000 rpm for 5 min. The supernatants were discarded, and 40 µL of 1 × PBS was added to each well to resuspend cells. 20 µL of cell suspension was taken and mixed with 20 µL of 0.2% trypan blue, and cells were counted. After the count, 100 µL (APC anti-human CD340 Antibody diluted with 1 × PBS at 1:100) of fluorescent antibody was added to each well of the co-culture group and well mixed, and the plate was then incubated in the dark at 2-8 °C for 15 min. After centrifugation at 1000 rpm for 5 min, the supernatants were discarded, and 200 µL of 1 × PBS was added to each well. The plate was centrifuged at 1000 rpm for 5 min and washed once, and the supernatants were pipetted off. 100 µL of 1 × PBS was added for resuspension, and a flow cytometry assay was performed.

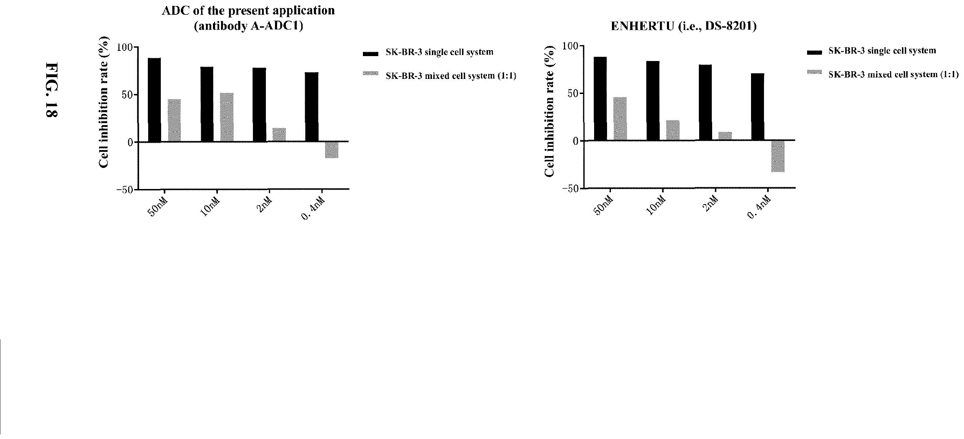

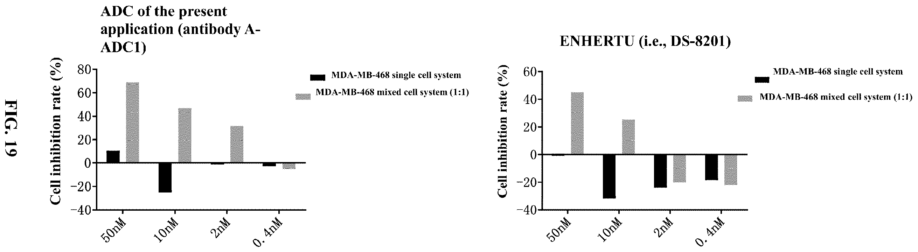

FIG. 18 shows the results of the inhibition of SK-BR-3 cell proliferation in each experimental group. FIG. 19 shows the results of the inhibition of MDA-MB-468 cell proliferation in each experimental group. The results show that: (a) both antibody A-ADC1 and ENHERTU (namely DS-8201) have bystander killing effects on the SK-BR-3 cells in the SK-BR-3 single cell system and the SK-BR-3 + MDA-MB-468 mixed system; (b) antibody A-ADC1 and ENHERTU did not produce significant killing effects on the HER2 negative MDA-MB-468 single cell system, but produced significant killing effects on the MDA-MB-468 cells in the mixed system. At the sample concentrations of 50 nM, 10 nM, 2 nM and 0.4 nM, the proliferation inhibition rates of antibody A-ADC1 against MDA-MB-468 cells in the mixed cell system are 68.65%, 46.87%, 31.61% and -4.95%, respectively; the proliferation inhibition rates of ENHERTU against MDA-MB-468 cells in the mixed cell system are 45.02%, 25.4%, -20.02% and -22.08%, respectively. At the concentration of 50 nM, the bystander killing ability of antibody A-ADC1 against HER2 negative cells is about 1.5 times that of ENHERTU. At the concentration of 10 nM, the bystander killing ability of antibody A-ADC1 against HER2 negative cells is about 1.8 times that of ENHERTU. At the concentration of 2 nM, antibody A-ADC1 still showed a bystander killing ability of 31.61%, while ENHERTU showed no bystander killing ability. Thus, at a concentration of 2-50 nmM, antibody A-ADC1 has a greater bystander killing ability than ENHERTU.

The table below shows the results of the CountStar cell count.

| Group | Antibody A-ADC1 | | | | ENHERTU (i.e., DS-8201) | | | | NC |
|---|---|---|---|---|---|---|---|---|---|
| | Concentration | | | | | | | | |
| | 50nM | 10nM | 2nM | 0.4nM | 50nM | 10nM | 2nM | 0.4nM | NA |
| SK-BR-3 single cell system | 1940 | 3640 | 3812 | 4680 | 2012 | 2808 | 3572 | 5120 | 17400 |
| MDA-MB-468 single cell system | 7800 | 10920 | 8840 | 8960 | 8800 | 11480 | 10800 | 10320 | 8720 |
| (SKBR-3 + MDA-MB-468) mixed system (1:1) | 9080 | 11240 | 16640 | 24200 | 12000 | 16760 | 23680 | 27860 | 21960 |

The table below shows the proportions of HER2 positive and HER2 negative cells in the mixed cell group.

| Group | Antibody A-ADC1 | | | | ENHERTU (i.e., DS-8201) | | | | NC |
|---|---|---|---|---|---|---|---|---|---|
| | Concentration | | | | | | | | |
| | 50nM | 10nM | 2nM | 0.4nM | 50nM | 10nM | 2nM | 0.4nM | NA |
| SK-BR-3 mixed system (1:1) | 56.41% | 40.31% | 48.10% | 45.24% | 42.15% | 43.80% | 36.00% | 44.67% | 42.50 % |
| MDA-MB-4 68 mixed system (1:1) | 43.59% | 59.69% | 51.90% | 54.76% | 57.85% | 56.20% | 64.00% | 55.33% | 57.50 % |

The table below shows the proliferation inhibition rates against SK-BR-3 cells.

| Inhibition rate (%) | | | | | |
|---|---|---|---|---|---|
| Group | Sample | Concentration | | | |
| | | 50nM | 10nM | 2nM | 0.4nM |
| SK-BR-3 single cell system | Antibody A-ADC1 | 88.85% | 79.08% | 78.09% | 73.10% |
| | ENHERTU (i.e., DS-8201) | 88.44% | 83.86% | 79.47% | 70.57% |
| SK-BR-3 mixed system (1:1) | Antibody A-ADC1 | 45.12% | 51.45% | 14.24% | -17.31% |
| | ENHERTU (i.e., DS-8201) | 45.81% | 21.34% | 8.66% | -33.34% |

The table below shows the proliferation inhibition rates against MDA-MB-468 cells.

| Inhibition rate (%) | | | | | |
|---|---|---|---|---|---|
| Group | Sample | Concentration | | | |
| | | 50nM | 10nM | 2nM | 0.4nM |
| MDA-MB-468 single cell system | Antibody A-ADC1 | 10.55% | -25.23% | -1.38% | -2.75% |
| | ENHERTU (i.e., DS-8201) | -0.92% | -31.65% | -23.85% | -18.35% |
| MDA-MB-468 mixed system (1:1) | Antibody A-ADC1 | 68.65% | 46.87% | 31.61% | -4.95% |
| | ENHERTU (i.e., DS-8201) | 45.02% | 25.40% | -20.02% | -22.08% |

### Results and conclusions

At the concentration of 50 nM, the bystander killing ability of antibody A-ADC1 against HER2 negative cells is about 1.5 times that of ENHERTU (i.e., DS-8201). At the concentration of 10 nM, the bystander killing ability of antibody A-ADC1 against HER2 negative cells is about 1.8 times that of ENHERTU. At the concentration of 2 nM, antibody A-ADC1 still showed a bystander killing ability of 31.61%, while ENHERTU showed no bystander killing ability. Thus, at a concentration of 2-50 nmM, antibody A-ADC1 has a greater bystander killing ability than ENHERTU.

### Example 7. Analysis of ADCC Activities of Antibody A-Containing ADC Molecules

### (a) Determination of ADCC activity of antibody A-ADC1 using reporter gene system

The target cell NCI-N87 and the effector cell Jurkat-FcyRIIIa-V158-NFAT were co-cultured with the antibody A-ADC1 prepared in Example 1 for 6 h, and the ADCC activity was determined by measuring the fluorescent RUL value.

### The specific steps of the experiment:

(1) Preparation of reaction medium: 2% FBS + 1640.
(2) Different concentrations of antibody A-ADC1, antibody A-ADC2 and antibody A were prepared using the reaction medium prepared in step (1), wherein the highest concentration was 15,000 ng/mL (final concentration: 10,000 ng/mL) and was 4-fold diluted to obtain 9 concentrations.
(3) NCI-N87 cells were collected, and the cell density was adjusted to 1 × 10⁶ cells/mL using the reaction medium prepared in step (1). Jurkat-FcyRIIIa-V158-NFAT cells were collected, and the cell density was adjusted to 4 × 10⁶ cells/mL using the reaction medium prepared in step (1).
(4) To a 96-well plate were added 25 µL of the antibody A or an antibody A-containing ADC prepared in step (2), 25 µL of N8 cells and 25 µL of Jurkat-FcyRIIIa-V158-NFAT cells, and the plate was left in an incubator for 6 h.
(5) 10 µL of Bio-Glo^{™} luciferase substrate was added to each well, and the RLU value of each well was immediately measured on a fluorescent microplate reader.

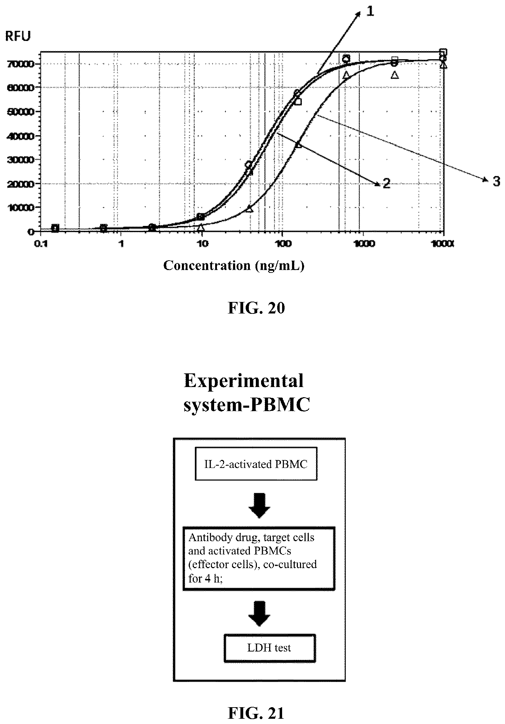

The results are shown in FIG. 20, where 1-3 show the results for antibody A, antibody A-ADC1 and antibody A-ADC2, respectively. The antibody A-ADC1 molecule prepared in Example 1 still maintained a good ADCC activity in HER2 positive/high HER2 expression cells (NCI-N87 cells), and the ADCC activity is 87.7% of that of the parent molecule antibody A (antibody A EC50/ADC1 EC50). The antibody A-ADC2 prepared in Example 2, on the other hand, lost most of the ADCC activity, and its activity is only 37.2% of that of the parent molecule antibody A (antibody A EC50/ADC2 EC50). This indicates that the way of conjugation affects the ADCC activities of the ADCs to a relatively great extent.

### (b) Determination of ADCC activities of antibody A-containing ADCs using PBMC System

### The specific steps of the assay (BT474 cells):

(1) 300 IU/mL PBMCs that had been activated with IL-2 for 24 h (medium for activation: 10% FBS + 1640 + 300 IU/mL) were collected, and the cell density was adjusted to 4.5 × 10⁶ cells/mL using 10% FBS + 1640 + 150 IU/mL.
(2) Different concentrations of antibody A-ADC1, antibody A-ADC2, DS8201 and human IgG1 were prepared using the medium for activation, wherein the highest concentration was 3 µg/mL (the final concentration was 1 µg/mL) and was 10-fold diluted to obtain 3 concentrations.
3. BT474 cells were collected, and the cell density was adjusted to 3 × 10⁵ cells/mL using the medium for activation.
4. To a 96-well plate were added 50 µL of an antibody A-containing ADC prepared in step (2), 50 µL of BT474 and 50 µL of PBMCs, and the plate was left in an incubator for 4 h.
5. 30 min before detection, 10 µL of cell lysis buffer was added to the TMR and VCC wells.
6. The plate was centrifuged at 1500 rpm for 5 min, and 50 µL of supernatant was transferred to a new 96-well plate. An LDH test substrate was added at 50 µL/well, and the plate was left at room temperature for 30 min.
7. OD values were measured at 490 nm.

### The specific steps of the assay (NCI-N87 cells):

(1) 300 IU/mL PBMCs that had been activated with IL-2 for 24 h (medium for activation: 10% FBS + 1640 + 300 IU/mL) were collected, and the cell density was adjusted to 4.5 × 10⁶ cells/mL using 10% FBS + 1640 + 150 IU/mL.
(2) Different concentrations of antibody A-ADC1, antibody A-ADC2, DS8201 and human IgG1 were prepared using the medium for activation, wherein the highest concentration was 3000 ng/mL (the final concentration was 1000 ng/mL) and was 6-fold diluted to obtain 5 concentrations.
3. NCI-N87 cells were collected, and the cell density was adjusted to 3 × 10⁵ cells/mL using the medium for activation.
4. To a 96-well plate were added 50 µL of an antibody A-containing ADC prepared in step (2), 50 µL of BT474 and 50 µL of PBMCs, and the plate was left in an incubator for 4 h.
5. 30 min before detection, 10 µL of cell lysis buffer was added to the TMR and VCC wells.
6. The plate was centrifuged at 1500 rpm for 5 min, and 50 µL of supernatant was transferred to a new 96-well plate. An LDH test substrate was added at 50 µL/well, and the plate was left at room temperature for 30 min.
7. OD values were measured at 490 nm.

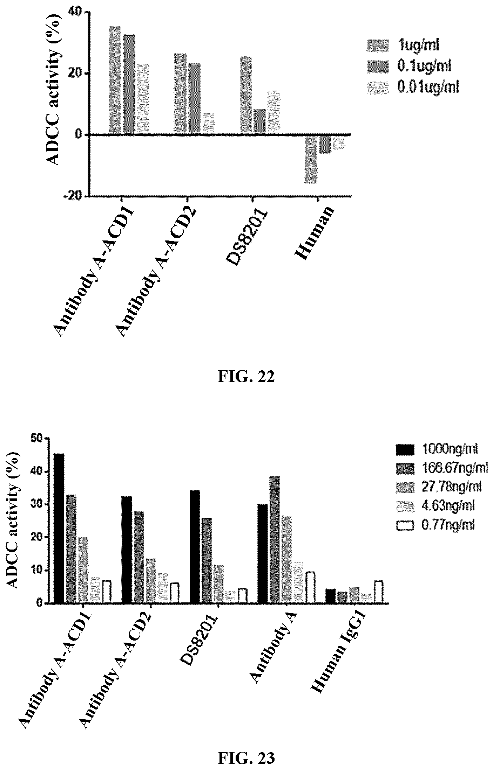

According to the steps of the method described above, the ADCC activities of antibody A-ADC1, antibody A-ADC2, DS8201, antibody A, etc. in a cell system highly expressing HER2 were determined. The target cells used were cells highly expressing HER2: BT747 and NCI-N87, and the effector cells were PBMCs (PBMC-qc and PBMC-z) in the peripheral blood of two donors. The detection process is shown in FIG. 21, and the detection results are shown in FIGs. 22-23.

The results show that antibody A-ADC1 showed better activities in the PBMC-qc: BT474 (effector-to-target ratio: 15:1) cell system and the PBMC-z: NCI-N87 (effector-to-target ratio: 15:1) cell system than antibody A-ADC2 and DS-8201, indicating that antibody A-ADC1 showed higher ADCC activities than DS-8201 in the cell systems highly expressing HER2. It can be seen that the ADCs of the present application retain the ADCC activity of the conjugated parent molecule antibody A.

### Example 8. Determination of Endocytosis Efficiencies of Antibody A-Containing ADC Molecules in Tumor Cells

The specific steps of the experiment (NCI-N87 cells, purchased from Chinese Academy of Sciences, Shanghai):
(1) Various concentrations of antibody A-ADC1, antibody A-ADC2, DS8201 and antibody A (mixed with an endocytosis reagent) were prepared using a 1640 medium containing 10% FBS: 50 µL of antibody A-ADC1, antibody A-ADC2, DS8201 or antibody A was added to a 96-well plate at final concentrations of 50 nM, 10 nM and 2 nM.
(2) NCI-N87 cells were digested, counted and added at 4 × 10⁴ cells/50 µL/drug-containing well. The plates were left in an incubator for 2 h, 5 h and 23 h.
(3) The cell supernatants in the 96-well plates were discarded, and the cells were digested with trypsin (50 µL/well) for 5 min.
(4) 200 µL of 1640 medium containing 10% FBS was added to each well for resuspension, and the cells were pipetted to form single-cell suspensions.
(5) The cell suspensions were transferred to 1.5-mL EP tubes and assayed by FACS.

### The specific steps of the experiment (BT474 cells):

(1) Various concentrations of antibody A-ADC1, antibody A-ADC2, DS8201 and antibody A (mixed with an endocytosis reagent) were prepared using a 1640 medium containing 10% FBS: 50 µL of antibody A-ADC1, antibody A-ADC2, DS8201 or antibody A was added to a 96-well plate at final concentrations of 40 nM, 8 nM and 1.6 nM.
(2) BT474 cells were digested, counted and added at 4 × 10⁴ cells/50 µL/drug-containing well. The plates were left in an incubator for 24 h.
(3) The cell supernatants in the 96-well plates were discarded, and the cells were digested with trypsin (50 µL/well) for 5 min.
(4) 200 µL of 1640 medium containing 10% FBS was added to each well for resuspension, and the cells were pipetted to form single-cell suspensions.
(5) The cell suspensions were transferred to 1.5-mL EP tubes and assayed by FACS.

According to the steps of the method described above, after antibody (such as antibody A-ADC1, antibody A-ADC2, DS-8201 or antibody A)-endocytosis reagent mixtures were incubated in the tumor cell systems, the efficiencies of the endocytosis of these molecules by the cells were determined by flow cytometry.

### (1) Endocytosis in NCI-N87 tumor cells

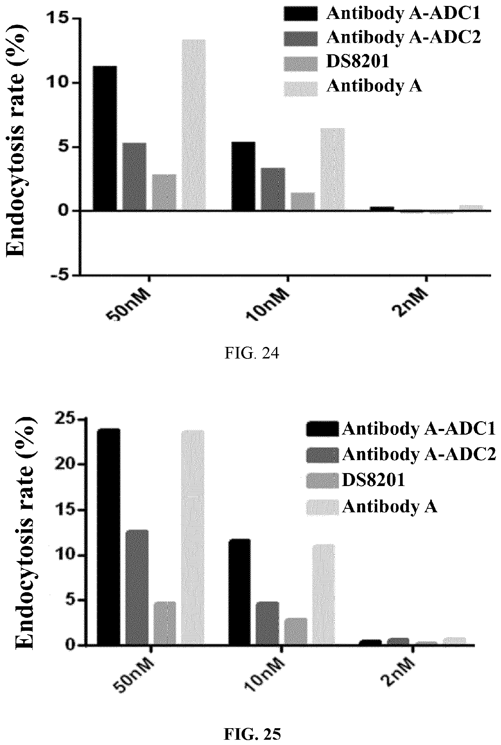

After the molecules antibody A-ADC1, antibody A-ADC2, DS-8201 and antibody A were incubated in the NCI-N87 cell system for 2 h, 5 h or 23 h, the endocytosis of these molecules by NCI-N87 cells was measured by flow cytometry. The results of the 2 h of incubation, 5 h of incubation and 23 h of incubation are shown in FIGs. 24, 25 and 26, respectively.
(a) At 2 h of incubation, the efficiencies of the endocytosis of these molecules by cells were in the following order: antibody A> antibody A-ADC1 > antibody A-ADC2 > DS-8201. At 5 h of incubation, the endocytosis efficiencies were in the following order: antibody A = antibody A-ADC1 >> antibody A-ADC2 >> DS-8201: the endocytosis efficiency of antibody A-ADC1 was equivalent to that of the parent molecule antibody A, and was far higher than those of DS-8201 and antibody A-ADC2. This indicates that after a short incubation (5 h), antibody A-ADC1 maintained an equivalent tumor cell endocytosis effect to the parent molecule and the effect was far better than that of DS-8201, suggesting that the ADCs of the present application can rapidly bind to HER2 on the tumor cell surface and achieve endocytosis, blocking the HER2 signaling pathway and producing tumor killing effects.
(b) At 23 h of incubation, the endocytosis efficiencies of antibody A-ADC1, antibody A-ADC2, antibody A, etc. were similar; at 50 nM, their endocytosis efficiencies all reached over 55% and were better than that of DS-8201, indicating that 55% of drug (50 nM) arrived in the NCI-N87 tumor target cells 23 h after the ADCs of the present application were administered: their targeting of tumors was slightly better than DS-8201's.

### (2) Endocytosis in BT474 tumor cells

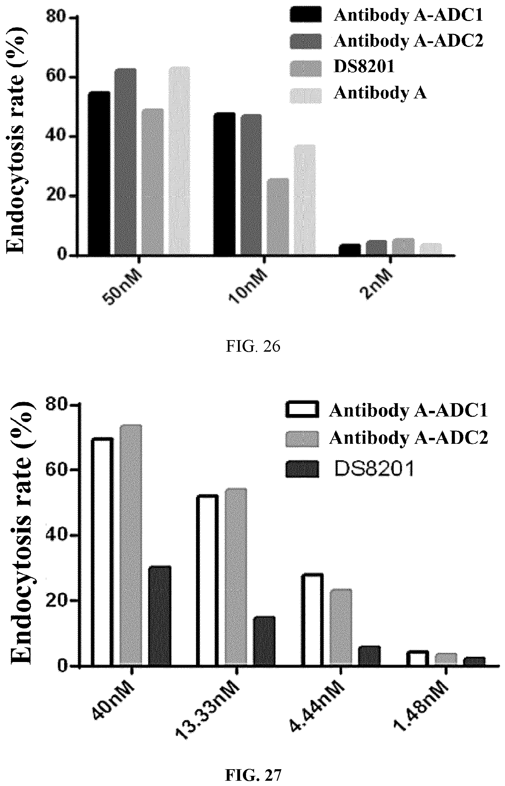

After antibody A-ADC1, antibody A-ADC2 and DS-8201 were incubated in the BT474 cell system for 24 h, a flow cytometry assay was performed. The results of the 24 h of incubation are shown in FIG. 27.

The results show that the endocytosis efficiencies of antibody A-ADC1 and antibody A-ADC2 were similar and reached over 70% at 40 nM, and it can be seen that the endocytosis efficiencies of the antibody A-containing ADCs of the present application are 2.33 times that of DS-8201 (30%) under equivalent conditions, and meanwhile, the ADCs' targeting of BT474 cells was far better than DS-8201's. This indicates that antibody A-ADC1 has a greater ability to inhibit target-mediated signaling pathways, a greater ability to kill tumors than DS-8201, and a greater ability to inhibit tumors.

### Example 9. Inhibition of Tumor Growth in Animal Models by Antibody A-Containing ADC Molecules

Group administration regimen: BxPC-3 cells were injected subcutaneously into the axilla of nude mice to establish a model, wherein the concentration for inoculation was 1 × 10⁶/0.1 mL/mouse. When the average tumor size reached about 190 mm³, the mice were randomly divided into groups of 4 (male) by tumor size and body weight. The day when random grouping was performed was taken as day 0. The test drugs were administered intraperitoneally on day 0, and administered intraperitoneally at the dose once every 3 days in the following days. A total of 2 administrations were performed. The animals not selected were euthanized with CO₂.

The test animals were divided into groups A, B and C. Group A was administered PBS, group B was administered antibody A-ADC1 (DAR = 4), and group C was administered DS-8201 (DAR = 8). The group administration regimen is specifically shown in Table 4.

**Table 4**

| Group | Route of administration | Number of administrations | Dose of test substance (mg/kg) | Volume of test substance (mL/kg) | Concentration of test substance (mg/mL) |
|---|---|---|---|---|---|
| Group A | Intraperitoneal injection | Twice a week for at least 24 days | - | 5 | - |
| Group B | | | 10 | | 2 |
| Group C | | | 10 | | 2 |

According to the above regimen, the test mice of groups A, B and C were put on three test substances for 24 days (2 animals from group A (PBS) were euthanized after 20 days of test substance administration because the tumors grew too big). Tumor inhibition curves of the three test substances against the BxPC-3 tumor model were obtained.

The tumor inhibition curves show that administering antibody A-ADC1 (DAR = 4) and DS-8201 (DAR = 8) to animals of the BxPC-3 tumor model caused tumor inhibition to a great extent, the inhibition of tumors became significant over time, and the inhibitory effects of antibody A-ADC1 and DS-8201 on tumors were similar.

### Example 10. Pharmacokinetic Study of Antibody A-Containing ADC Molecules in Balb/c-Nu Nude Mouse NCI-N87 Tumor Model

An NCI-N87 tumor model was established by subcutaneously inoculating Balb/c-Nu nude mice with the human gastric cancer cell NCI-N87, and animals of the model were intravenously (iv) administered A-ADC1 1 mg/kg and ENHERTU (i.e., DS-8201) 1 mg/kg. Blood samples were collected at different time points, and the animal serum samples were assayed by Elisa for total antibody and ADC contents (method 1: ELISA assay for total antibody content, including ADCs and naked antibodies; method 2: ELISA assay for ADC content (including ADCs containing 1-4 drugs), excluding naked antibodies formed after toxin release). A non-compartmental model of DAS (3.2.8) software was used to calculate pharmacokinetic parameters.

**Table 5**

| Group | Number of animals | Test substance | Dose (mg/kg) | Administration concentration (mg/mL) | Administration volume (mL/kg) | Solvent | Route of administration | Sample collected | Blood collection time points |
|---|---|---|---|---|---|---|---|---|---|
| | Female | | | | | | | | |
| 1 | Each group contained 8 mice: Blood collection time points for mice 1-4: before administration, 2 min, 6 h, 48 h (2 days), and 168 h (7 days); blood collection time points for mice 5-8: before administration, 1 h, 24 h (1 day), and 96 h (4 days); | Antibody A-ADC1 | 1 | 0.2 | 5 | PBS | IV | Serum | Collecting blood samples of about 100 µL from the orbit, before administrati on, and at 2 min, 1 h, 6 h, 24 h (1 day), 48 h (2 days), 96 h (4 days), and 168 h (7 days). |
| 2 | | ENHERTU (i.e., DS-8201) | 1 | 0.2 | | | | | |

After single intravenous injections of antibody A-ADC1 into mice of the NCI-N87 tumor model, for total antibody and ADC, the Cₘₐₓ values were 45,422.727 µg/L and 70,056.81675 µg/L, respectively; the AUC₍₀₋ₜ₎ values were 1,598,292.175 µg/L*h and 2,098,916.987 µg/L*h, respectively; the Tₘₐₓ values were 0.033 h and 0.033 h, respectively; and the T_{1/2} values were 100.976 h and 95.32 h, respectively.

After single intravenous injections of ENHERTU (i.e., DS-8201) into mice of the NCI-N87 tumor model, for total antibody and ADC, the Cₘₐₓ values were 12,343.966 µg/L and 19,604.51225 µg/L, respectively; the AUC₍₀₋ₜ₎ values were 421,103.345 µg/L*h and 586,509.313 µg/L*h, respectively; the Tₘₐₓ values were 0.033 h and 0.033 h, respectively; and the T_{1/2} values were 86.744 h and 65.384 h, respectively.

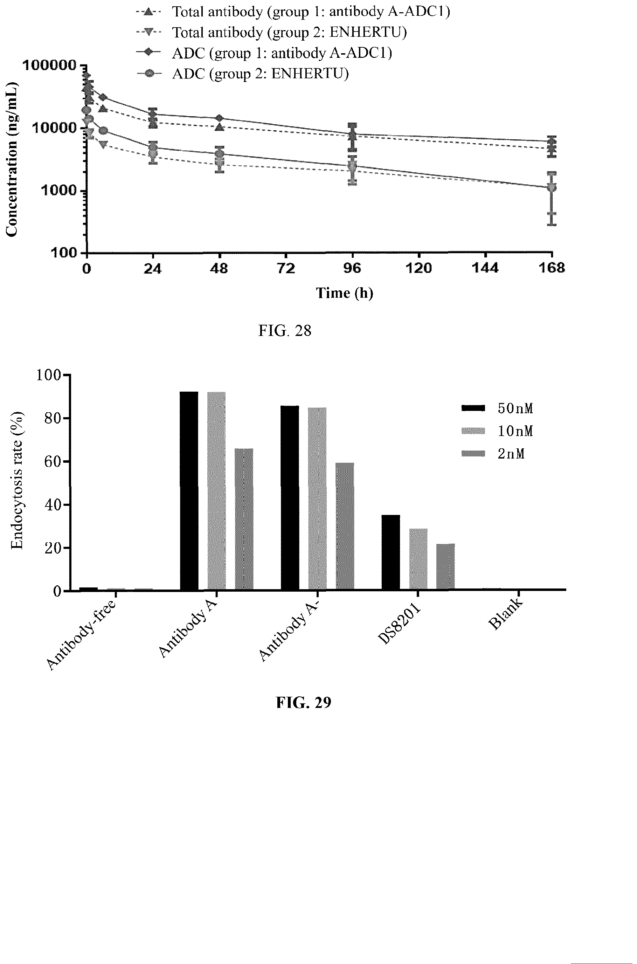

In summary, after single intravenous injections of antibody A-ADC1 and ENHERTU into mice of the NCI-N87 tumor model, the results for Elisa method 1 (total antibody concentration) show that the T_{1/2} values were 100.976 h and 86.744 h, respectively; the T_{1/2} of antibody A-ADC1 is 1.164 times that of ENHERTU, the Cmax of antibody A-ADC1 is 3.680 times that of ENHERTU, and the AUC₍₀₋ₜ₎ of antibody A-ADC1 is 3.795 times that of ENHERTU. The results for method 2 (antibody-drug conjugate content) show that the T_{1/2} values of antibody A-ADC1 and ENHERTU were 95.32 h and 65.384 h, respectively; the T_{1/2} of antibody A-ADC1 is 1.460 times that of ENHERTU, the Cmax of antibody A-ADC1 is 3.574 times that of ENHERTU, and the AUC₍₀₋ₜ₎ of antibody A-ADC1 is 3.580 times that of ENHERTU. The results can be seen in FIG. 28.

### Example 11. Effects of Antibody A-Containing ADC Molecules on Endocytosis of NCI-N87 Cell Surface HER2

Her2-targeting monoclonal antibodies can promote Her2 internalization degradation to further inhibit downstream signaling of Her2, thereby inhibiting cell proliferation. The ADC of the present application (antibody A-ADC1) is a HER2-targeting bi-epitopic antibody, for example, an ADC drug formed by conjugating the small molecule DXd to antibody A. Human gastric cancer cell line NCI-N87 cells overly expressing Her2 were selected to compare the endocytosis effects of antibody A-ADC1, antibody A, and DS8201, which is a commercially available drug of the same target.

### The steps of the experiment:

1) An experimental buffer was prepared: 1640 + 10% FBS medium.
2) After N87 cells were centrifuged at 350 g for 5 min, the supernatant was removed, and the density was adjusted to 1 × 106 cells/mL using the experimental buffer.
3) The test antibody was diluted with the experimental buffer to an initial concentration of 200 nM, and pHrodo was diluted with the experimental buffer to a concentration of 600 nM.
4) The 200 nM antibody and 600 nM pHrodo obtained above were mixed at 1:1 and incubated for 15 min, and the mixture of the antibody and pHrodo was then 5-fold diluted with the experimental buffer to a total of 3 concentration points. After 5 min of incubation, 50 µL of cells was added.
5) To sample wells were added 50 µL of the antibody-pHrodo mixture and 50 µL of cells.
6) NC well: 25 µL of Buffer + 25 µL of PHrodo + 50 µL of cells (1E5).
7) BLANK well: 50 µL of Buffer + 50 µL of cells (1E5). The volume in each well was 100 µL. The contents in each well were pipetted and well mixed.
8) The plate was incubated in a 37 °C cell incubator for 5 h.
9) After the incubation was complete, the plate was centrifuged at 350× g for 5 min, and the supernatants were removed. Trypsin was added to each well to digest the cells, and the cells were resuspended in the medium to form single-cell suspensions. The plate was centrifuged at 350× g for 5 min, and 1× PBS was added at 200 µL/well for resuspension.
10) After centrifugation at 350× g for 5 min, 1× PBS was added at 150 µL/well to resuspend the cells, and the cells were transferred to a new 96-well plate and assayed by flow cytometry.

### The results of the experiment:

Antibody A, antibody A-ADC1 and DS8201 all induced endocytosis of NCI-N87 cell surface HER2, and antibody A-ADC1 and antibody A produced similar endocytosis effects. In addition, the endocytosis effects of antibody A-ADC1 were significantly stronger than those of DS8201 at the three concentration points: at the concentrations of 50 nM, 10 nM and 2 nM, the endocytosis ratios of antibody A were 92.25%, 91.82% and 65.71%, respectively; the endocytosis ratios of antibody A-ADC1 were 85.48%, 84.65% and 58.95%, respectively; and the endocytosis ratios of DS8201 were 34.79%, 28.36% and 21.26%, respectively. The endocytosis effect of antibody A-ADC1 on NCI-N87 was similar to that of antibody A and was significantly stronger than that of DS8201.

**Table 6**

| Sample concentration | Proportion of positive cells | | | | |
|---|---|---|---|---|---|
| | Antibody-free | Antibody A | Antibody A-ADC1 | DS8201 | Blank group |
| 50nM | 1.72% | 92.25% | 85.48% | 34.79% | 0.70% |
| 10nM | 1.29% | 91.82% | 84.65% | 28.36% | |
| 2nM | 1.06% | 65.71% | 58.95% | 21.26% | |

Table 6 shows the proportions of positive NCI-N87 cells after endocytosis of antibody A, antibody A-ADC1 and DS8201.

FIG. 29 shows the proportions of NCI-N87 positive cells after endocytosis induced by antibody A, antibody A-ADC1 and DS8201.

### Example 12. Inhibitory Effects of Antibody A-Containing ADC Molecules on HER2 Weakly Positive Gastric Cancer PDX Subcutaneous Xenograft NOD/SCID Mouse Model

The anti-tumor effect of the ADC of the present application (antibody A-ADC1) was assessed in a human HER2 weakly positive (2+) gastric cancer PDX subcutaneous xenograft NOD/SCID mouse model and compared with that of the commercially available positive control drug ENHERTU (i.e., DS-8201).

### Model information

The table below shows the sample information about the human gastric cancer PDX xenograft model.

| Model ID | Type of cancer | Race | Characteristics |
|---|---|---|---|
| Case168 | Gastric adenocarcinoma | Asian | HER2 (2+) P6 |

### The process of the experiment

For each case of the PDX model, 30 four-to-five-week-old NOD/SCID female mice were selected and subcutaneously inoculated with gastric cancer PDX tumors with a diameter of 2-3 mm in the right anterior scapular region. When the average tumor volume reached 213.40 mm³, the mice were randomly divided into groups by tumor size and body weight (see the table below). The day when grouping was performed was defined as day 1. After the grouping, administration was started immediately. The day when administration was performed was defined as day 1 after administration.

The table below shows the experimental design for testing the antitumor effect of the drug in the human gastric cancer PDX tumor model.

| Group | Number of animals | Administration group | Dose | Administration volume | Route of administration | Planed administration cycle | Actual administration cycle |
|---|---|---|---|---|---|---|---|
| Negative control group | 5 | PBS | -- | 10 µL/g | Tail vein injection | QW × 3 weeks | A total of two administrations were performed: the first day after the first group administration (day 1), and the 10th day after the first group administration (day 10) |
| Test drug group | 5 | ADC of the present application (antibody A-ADC1) | 10 mg/kg | 10 µL/g | Tail vein injection | QW × 3 weeks | |
| Positive control group | 5 | ENHERTU (i.e., DS-8201) | 10 mg/kg | 10 µL/g | Tail vein injection | QW × 3 weeks | |

### The results of the experiment:

On day 27 after the first administration (at that time, the longest diameter of the tumor of the control group had reached 1.5 cm, the upper limit permitted by the ethics of mouse experimentation), the animals of the control group and the test drug group were all alive. Thus, the tumor volume data at that time were selected for the analysis and assessment of the anti-tumor effect of the test drug, the ADC of the present application (antibody A-ADC1). On day 27 after the first group administration, the average tumor volume of the mice in the PBS control group (CTL) was 725.39 ± 190.25 mm³, and the relative tumor volume was 3.58 ± 1.11.

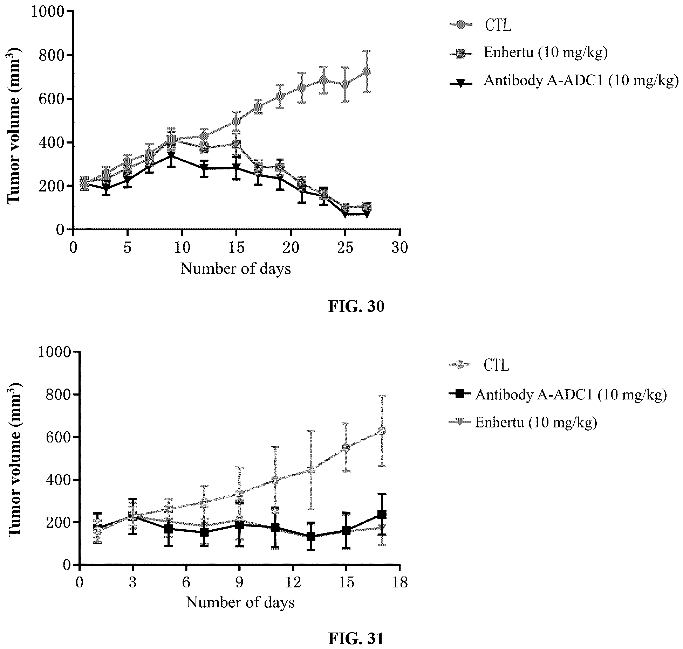

On day 27 after the first group administration, the average tumor volume of the test drug antibody A-ADC1 (10 mg/kg) group was 112.89 ± 61.65 mm³, the relative tumor volume was 0.50 ± 0.16, and the relative tumor inhibition rate TGI (%) was 86.05%: there was a statistically significant difference (p < 0.0001) from the PBS control group. On day 27 after the first group administration, the average tumor volume of the positive control drug ENHERTU (i.e., DS-8201) (10 mg/kg) group was 106.41 ± 17.77 mm³, the relative tumor volume was 0.50 ± 0.11, and the relative tumor inhibition rate TGI (%) was 86.08%: there was a statistically significant difference (p < 0.0001) from the PBS control group. Moreover, at the dose of 10 mg/kg, the average tumor volume of the test drug antibody A-ADC1 treatment group was similar to that of the ENHERTU treatment group on day 27 after the first group administration, and there was no statistically significant difference (antibody A-ADC1 VS ENHERTU, P = 0.8362 > 0.05, ns.). In general, at the dose of 10 mg/kg, the antibody A-ADC1 showed a significant tumor inhibition effect on the human HER2 IHC 2+ gastric cancer PDX tumor model; antibody A-ADC1 with a DAR of 4 showed a similar anti-tumor effect to the positive control drug ENHERTU with a DAR of 8. FIG. 30 shows tumor volume growth curves of these groups of mice of the human gastric cancer PDX tumor model.

The table below shows a drug effect analysis for each group in case 168 of the human gastric cancer PDX tumor model.

| Experimental group | Day 27 after the first administration | | | | |
|---|---|---|---|---|---|
| | Tumor volume (mm³) (*x̅* ± S) | Relative tumor volume | T/C (%) | TGI (%) | P value (relative to control group CTL) |
| CTL (PBS) | 725.39 ± 190.25 | 3.58± 1.11 | -- | -- | -- |
| Antibody A-ADC1 (10 mg/kg) | 112.89 ± 61.65 | 0.50 ± 0.16 | 13.95 | 86.05 | < 0.0001 |
| ENHERTU (i.e., DS-8201) (10 mg/kg) | 106.41 ± 17.77 | 0.50 ± 0.11 | 13.92 | 86.08 | < 0.0001 |

| | | | | | |
|---|---|---|---|---|---|
| Notes: 1. Data are expressed as "mean (*x̅*) ± standard error (S)"; 2. T/C % = T_{RTV}/C_{RTV} × 100%; TGI% = (1 - T_{RTV}/C_{RTV}) × 100%. 3. CTL VS the ADC of the present application (antibody A-ADC1), P < 0.0001; CTL VS ENHERTU (i.e., DS-8201), P < 0.0001; antibody A-ADC1 VS ENHERTU, P = 0.8362 > 0.05. | | | | | |

### Example 13. Inhibitory Effects of Antibody A-Containing ADC Molecules on HER2 Negative Gastric Cancer PDX Subcutaneous Xenograft NOD/SCID Mouse Model

The anti-tumor effect of the ADC of the present application (antibody A-ADC1) was assessed in a HER2 expression negative human gastric cancer PDX tumor subcutaneous xenograft NOD/SCID mouse model and compared with that of the commercially available positive control drug ENHERTU (i.e., DS-8201).

### The process of the experiment:

### Model information

The table below shows the sample information about the human gastric cancer HER2 IHC 2+ PDX xenograft model.

| Model ID | Type of cancer | Race | Characteristics |
|---|---|---|---|
| Case 111 | Gastric adenocarcinoma | Asian | HER2 (-) P6 |

### The process of the experiment

For each case of the PDX model, 30 four-to-five-week-old female NOD/SCID mice were selected and inoculated with gastric cancer PDX tumors with a diameter of 2-3 mm in the right anterior scapular region. When the average tumor volume reached 166.53 mm³, the mice were randomly divided into groups by tumor size and body weight. The day when grouping was performed was defined as day 1. After the grouping, administration was started immediately. The day when administration was performed was defined as day 1 after administration.

The table below shows the experimental design for testing the antitumor effect of the drug in the human gastric cancer PDX tumor model.

| Group | Number of animals | Administration group | Dose | Administration volume | Route of administration | Actual administration cycle |
|---|---|---|---|---|---|---|
| Negative control group | 5 | PBS | -- | 10 µL/g | Tail vein injection | Administration was performed only once: the 1st day after the first group administration (day 1) |
| Test drug group | 5 | Antibody A-ADC1 | 10 mg/kg | 10 µL/g | Tail vein injection | |
| Positive control group | 5 | ENHERTU (i.e., DS-8201) | 10 mg/kg | 10 µL/g | Tail vein injection | |

### The results of the experiment:

On day 17 after the first group administration (at that time, the longest diameter of the tumor of the control group had reached 1.5 cm, the upper limit permitted by the ethics of mouse experimentation), the animals of the control group and the test drug group were all alive. Thus, the tumor volume data at that time were selected for the assessment of the anti-tumor effect of the test drug, the ADC of the present application (antibody A-ADC1). On day 17 after the first group administration, the average tumor volume of the mice in the PBS control group was 629.24 ± 146.59 mm³, and the relative tumor volume was 4.13 ± 0.97. On day 17 after the first group administration, the average tumor volume of the test drug antibody A-ADC1 (10 mg/kg) group was 238.32 ± 84.57 mm³, the relative tumor volume was 1.68 ± 1.01, and the relative tumor inhibition rate TGI (%) was 59.41%: there was a statistically significant difference (p < 0.0001) from the PBS control group. On day 17 after the first group administration, the average tumor volume of the positive control drug ENHERTU (i.e., DS-8201) (10 mg/kg) group was 174.06 ± 71.09 mm³, the relative tumor volume was 1.02 ± 0.34, and the relative tumor inhibition rate TGI (%) was 75.19%: there was a statistically significant difference (p < 0.0001) from the PBS control group. Moreover, at the dose of 10 mg/kg, the average tumor volume of the test drug antibody A-ADC1 treatment group was slightly greater than that of the positive control drug ENHERTU treatment group on day 17 after the first group administration, but there was no statistically significant difference (antibody A-ADC1 VS ENHERTU, P = 0.526 > 0.05, ns.). In general, at the dose of 10 mg/kg, the antibody A-ADC1 showed a significant tumor inhibition effect on the human HER2 - gastric cancer PDX tumor model; antibody A-ADC1 with a DAR of 4 showed a similar anti-tumor effect to the positive control drug ENHERTU with a DAR of 8. FIG. 31 shows tumor volume growth curves of mice of the human gastric cancer HER2 IHC- PDX tumor model (treated once with the drug on day 1 only, tail vein injection, 10 mg/kg).

The table below shows a drug effect analysis for each group in case 111 of the human gastric cancer PDX tumor model.

| Experimental group | Day 17 after the first administration | | | | |
|---|---|---|---|---|---|
| | Tumor volume (mm³) (*x̅* ± S) | Relative tumor volume | T/C (%) | TGI (%) | P value (relative to control group) |
| CTL (PBS) | 629.24 ± 146.59 | 4.13 ± 0.97 | -- | -- | -- |
| Antibody A-ADC1 (10 mg/kg) | 238.32 ± 84.57 | 1.68 ± 1.01 | 40.59 | 59.41 | < 0.0001 |
| ENHERTU (i.e., DS-8201) (10 mg/kg) | 174.06 ±71.09 | 1.02 ± 0.34 | 24.81 | 75.19 | < 0.0001 |

| | | | | | |
|---|---|---|---|---|---|
| Notes: 1. Data are expressed as "mean (*x̅*) ± standard error (S)"; 2. T/C % = T_{RTV}/C_{RTV} × 100%; TGI% = (1 - T_{RTV}/C_{RTV}) ^{×} 100%. 3. CTL VS the ADC of the present application (antibody A-ADC1), P < 0.0001; CTL VS ENHERTU (i.e., DS-8201), P < 0.0001; antibody A-ADC1 VS ENHERTU, P = 0.526 > 0.05, ns. | | | | | |

The foregoing detailed description is provided by way of illustration and example, and is not intended to limit the scope of the appended claims. Various variants of the embodiments currently listed in the present application will be apparent to those of ordinary skill in the art, and are intended to be within the scope of the appended claims and equivalents thereof.

## Claims

1. An antibody-drug conjugate, comprising a HER2-targeting bispecific antibody or an antigen-binding fragment thereof.

2. The antibody-drug conjugate according to claim 1, wherein the HER2-targeting bispecific antibody or the antigen-binding fragment thereof specifically binds to at least one epitope of human HER2.

3. The antibody-drug conjugate according to any one of claims 1-2, wherein the HER2-targeting bispecific antibody or the antigen-binding fragment thereof specifically binds to extracellular domain II of human HER2 and/or extracellular domain IV of human HER2.

4. The antibody-drug conjugate according to any one of claims 1-3, wherein the HER2-targeting bispecific antibody or the antigen-binding fragment thereof comprises a first light chain and a second light chain.

5. The antibody-drug conjugate according to claim 4, wherein the first light chain comprises first LCDR1-3, wherein the first LCDR1 comprises an amino acid sequence set forth in SEQ ID NO: 4.

6. The antibody-drug conjugate according to claim 5, wherein the first LCDR2 comprises an amino acid sequence set forth in SEQ ID NO: 5.

7. The antibody-drug conjugate according to any one of claims 5-6, wherein the first LCDR3 comprises an amino acid sequence set forth in SEQ ID NO: 6.

8. The antibody-drug conjugate according to any one of claims 4-7, wherein the first light chain is capable of binding to the heavy chain of pertuzumab.

9. The antibody-drug conjugate according to any one of claims 4-8, wherein the second light chain comprises second LCDR1-3, wherein the second LCDR1 comprises an amino acid sequence set forth in SEQ ID NO: 1.

10. The antibody-drug conjugate according to claim 9, wherein the second LCDR2 comprises an amino acid sequence set forth in SEQ ID NO: 2.

11. The antibody-drug conjugate according to any one of claims 9-10, wherein the second LCDR3 comprises an amino acid sequence set forth in SEQ ID NO: 3.

12. The antibody-drug conjugate according to any one of claims 4-11, wherein the second light chain is capable of binding to the heavy chain of trastuzumab.

13. The antibody-drug conjugate according to any one of claims 4-12, wherein a variable region of the first light chain and the second light chain comprises an amino acid sequence set forth in any one of SEQ ID NOs: 7-12.

14. The antibody-drug conjugate according to any one of claims 4-13, wherein a variable region of the first light chain and the second light chain comprises an amino acid sequence set forth in SEQ ID NO: 7.

15. The antibody-drug conjugate according to any one of claims 4-14, wherein the first light chain and the second light chain comprise an amino acid sequence set forth in any one of SEQ ID NOs: 13-18.

16. The antibody-drug conjugate according to any one of claims 4-15, wherein the first light chain is selected from the group consisting of the light chain of pertuzumab or a mutant thereof and the light chain of trastuzumab or a mutant thereof; and/or, the second light chain is selected from the group consisting of the light chain of pertuzumab or a mutant thereof and the light chain of trastuzumab or a mutant thereof.

17. The antibody-drug conjugate according to any one of claims 4-16, wherein amino acid sequences of the first light chain and the second light chain are identical.

18. The antibody-drug conjugate according to any one of claims 4-17, wherein the first light chain and the second light chain comprise an amino acid sequence set forth in SEQ ID NO: 13.

19. The antibody-drug conjugate according to any one of claims 1-18, wherein the HER2-targeting bispecific antibody or the antigen-binding fragment thereof comprises a first heavy chain and a second heavy chain, wherein the first heavy chain is capable of properly binding to the first light chain under physiological conditions or in an *in vitro* protein expression state.

20. The antibody-drug conjugate according to claim 19, wherein the second heavy chain is capable of properly binding to the second light chain under physiological conditions or in an *in vitro* protein expression state.

21. The antibody-drug conjugate according to any one of claims 19-20, wherein the first heavy chain comprises a first heavy chain variable region, and the first heavy chain variable region is the heavy chain variable region of pertuzumab.

22. The antibody-drug conjugate according to any one of claims 19-21, wherein the second heavy chain comprises a second heavy chain variable region, and the second heavy chain variable region is the heavy chain variable region of trastuzumab.

23. The antibody-drug conjugate according to any one of claims 19-22, wherein the first heavy chain and the second heavy chain comprise heavy chain constant regions, wherein the heavy chain constant regions are derived from the constant region of a human IgG.

24. The antibody-drug conjugate according to any one of claims 19-23, wherein the Fc fragment of the first heavy chain and the second heavy chain comprises an amino acid sequence set forth in any one of SEQ ID NOs: 25-57.

25. The antibody-drug conjugate according to any one of claims 19-24, wherein the first heavy chain comprises an amino acid sequence set forth in SEQ ID NO: 21 or 23.

26. The antibody-drug conjugate according to any one of claims 19-25, wherein the second heavy chain comprises an amino acid sequence set forth in SEQ ID NO: 22 or 24.

27. The antibody-drug conjugate according to any one of claims 1-26, comprising a structure represented by formula 1:
M-(L1)ₐ-(L2)_{b}-D (formula 1),
wherein M represents the HER2-targeting bispecific antibody or the antigen-binding fragment thereof according to any one of claims 1-26;
L1 represents a linker linking to M, and L2 represents a linker linking to D;
a and b are each independently selected from 0-10;
D represents a drug.

28. The antibody-drug conjugate according to claim 27, wherein the L1 and/or L2 are/is selected from the group consisting of: a cleavable linker, a non-cleavable linker, a hydrophilic linker, a hydrophobic linker, a charged linker, an uncharged linker, and a dicarboxylic acid-based linker.

29. The antibody-drug conjugate according to any one of claims 27-28, wherein the L1 and the M are linked by a sulfhydryl, azido or amide group on the M.

30. The antibody-drug conjugate according to any one of claims 27-29, wherein the M comprises a first heavy chain and a second heavy chain, and the first heavy chain and/or the second heavy chain comprise(s) a linking site capable of linking to the L1.

31. The antibody-drug conjugate according to claim 30, wherein the linking site comprises a group capable of linking to the L1 following deglycosylation modification.

32. The antibody-drug conjugate according to claim 31, wherein the group is located at the side group of the amino acid Q at position 297 of the first heavy chain; and/or is located at the side group of the amino acid Q at position 298 of the second heavy chain.

33. The antibody-drug conjugate according to claim 30, wherein the linking site comprises a group capable of linking to the L1 following glycosylation modification.

34. The antibody-drug conjugate according to claim 33, wherein the group is located at the side group of the amino acid N at position 299 of the first heavy chain; and/or is located at the side group of the amino acid N at position 300 of the second heavy chain.

35. The antibody-drug conjugate according to any one of claims 33-34, wherein the group comprises -N₃.

36. The antibody-drug conjugate according to any one of claims 33-35, wherein the glycosylation modification comprises: the M having been contacted with UDP-GalNAz, β-1,4-galactosyltransferase or a variant thereof.

37. The antibody-drug conjugate according to any one of claims 27-36, wherein the L1 is capable of taking part in a SPAAC reaction.

38. The antibody-drug conjugate according to any one of claims 27-37, wherein the L1 is selected from the group consisting of: maleimide, succinimide-3-yl-N, and DBCO.

39. The antibody-drug conjugate according to any one of claims 27-38, wherein the L1 is DBCO-(PEG)ₙ₁, wherein ₙ₁ is an integer from 0-10, or the L1 is maleimide.

40. The antibody-drug conjugate according to any one of claims 27-39, wherein the L2 is selected from the group consisting of: a polypeptide, VC-PAB, N-succinimidyl-3-(2-pyridyldithio)propionate (SPDP), N-succinimidyl 4-(2-pyridyldithio)valerate (SPP), N-succinimidyl 4-(2-pyridyldithio)butyrate (SPDB), N-succinimidyl-4-(2-pyridyldithio)-2-sulfobutyrate (sulfo-SPDB), N-succinimidyl iodoacetate (SIA), N-succinimidyl (4-iodoacetyl)aminobenzoate (SIAB), maleimide PEG NHS, N-succinimidyl 4-(maleimidomethyl)cyclohexylcarboxylate (SMCC), sulfosuccinimidyl 4-(N-maleimidomethyl)cyclohexane-1-carboxylate (sulfo-SMCC) and 2,5-dioxopyrrolidin-1-yl 17-(2,5-dioxo-2,5-dihydro-1H-pyrrol-1-yl)-5,8,11,14-tetraoxy-4,7,10,13-tetraazaoctadecan-1-oate (CX1-1).

41. The antibody-drug conjugate according to any one of claims 27-40, wherein the L2 is GGFG.

42. The antibody-drug conjugate according to any one of claims 27-41, wherein the drug has an ability to kill tumor cells, and/or an ability to inhibit tumor cell growth.

43. The antibody-drug conjugate according to any one of claims 27-42, wherein the drug includes small-molecule drugs.

44. The antibody-drug conjugate according to any one of claims 27-43, wherein the drug is selected from the group consisting of: a V-ATPase inhibitor, a Bcl2 inhibitor, an MCL1 inhibitor, an HSP90 inhibitor, an IAP inhibitor, an mTor inhibitor, a microtubule stabilizer, a microtubule destabilizer, auristatin, dolastatin, a maytansinoid, MetAP (methionine aminopeptidase), an inhibitor of nuclear export of protein CRM1, a DPPIV inhibitor, a proteasome inhibitor, an inhibitor of phosphoryl transfer reactions in mitochondria, a protein synthesis inhibitor, a CDK2 inhibitor, a CDK9 inhibitor, a kinesin inhibitor, an HDAC inhibitor, a DNA damaging agent, a DNA alkylating agent, a DNA intercalator, a DNA minor groove binder, a DHFR inhibitor, a nucleoside analog, an HD AC inhibitor, an anthracycline, a NAMPT inhibitor, SN-38 glucuronic acid, etoposide phosphate, nitrogen mustard, a proteosome inhibitor, a cytokine, and a Toll-like receptor agonist.

45. The antibody-drug conjugate according to any one of claims 27-44, wherein the drug is selected from the group consisting of: DM1, exatecan, DXd, MMAE, SN-38, calicheamicin, anthracyclin-5G, DM4, microtubule inhibitor SHR153024, PNU-159682, Duo5 toxin, an SN38 derivative or derivatives thereof.

46. The antibody-drug conjugate according to any one of claims 27-45, having a structure selected from the group consisting of: 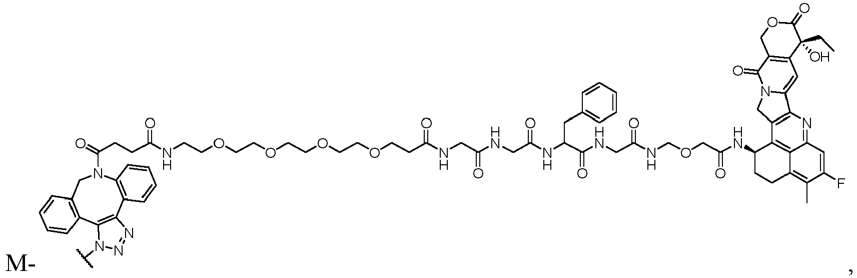 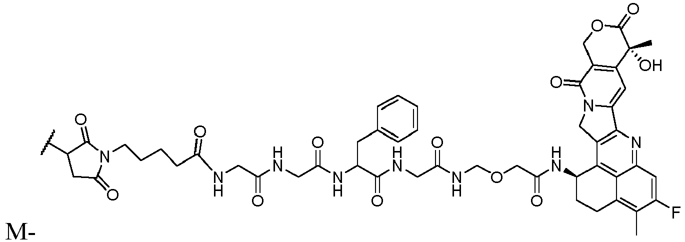 and

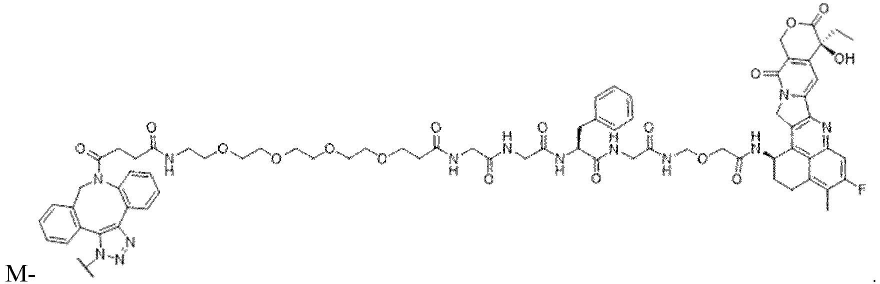

47. The antibody-drug conjugate according to any one of claims 1-46, having a drug/antibody ratio of about 1-6.

48. A compound for preparing the antibody-drug conjugate according to any one of claims 1-47, having a structure represented by formula 2:
M-(L1)ₐ (formula 2),
wherein M represents the HER2-targeting bispecific antibody or the antigen-binding fragment thereof according to any one of claims 1-26;
L1 represents a linker linking to M;
a is selected from 0-10.

49. The compound according to claim 48, wherein the L1 is selected from the group consisting of: a cleavable linker, a non-cleavable linker, a hydrophilic linker, a hydrophobic linker, a charged linker, an uncharged linker, and a dicarboxylic acid-based linker.

50. The compound according to any one of claims 48-49, wherein the L1 and the M are linked by a sulfhydryl, azido or amide group on the M.

51. The compound according to any one of claims 48-50, wherein the M comprises a first heavy chain and a second heavy chain, and the first heavy chain and/or the second heavy chain comprise(s) a linking site capable of linking to the L1.

52. The compound according to claim 51, wherein the linking site comprises a group capable of linking to the L1 following deglycosylation modification.

53. The compound according to claim 51, wherein the group is located at the side group of the amino acid Q at position 297 of the first heavy chain; and/or is located at the side group of the amino acid Q at position 298 of the second heavy chain.

54. The compound according to claim 51, wherein the linking site comprises a group capable of linking to the L1 following glycosylation modification.

55. The compound according to claim 54, wherein the group is located at the side group of the amino acid N at position 299 of the first heavy chain; and/or is located at the side group of the amino acid N at position 300 of the second heavy chain.

56. The compound according to any one of claims 54-55, wherein the group comprises -N₃.

57. The compound according to any one of claims 53-56, wherein the glycosylation modification comprises: the M having been contacted with UDP-GalNAz, β-1,4-galactosyltransferase or a variant thereof.

58. The compound according to any one of claims 48-57, wherein the L1 is capable of taking part in a SPAAC reaction.

59. The compound according to any one of claims 48-58, wherein the L1 is selected from the group consisting of: maleimide, succinimide-3-yl-N, and DBCO.

60. The compound according to any one of claims 48-59, wherein the L1 is DBCO-(PEG)ₙ₁, wherein ₙ₁ is an integer from 0-10, or the L1 is maleimide.

61. The compound according to any one of claims 48-60, comprising a structure selected from the group consisting of:

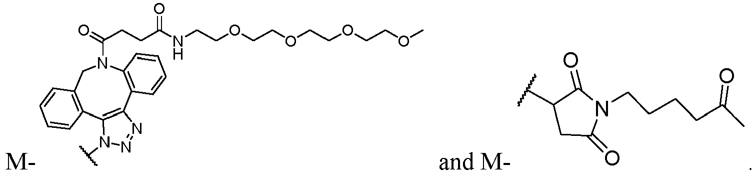

62. A method for preparing the antibody-drug conjugate according to any one of claims 1-47, comprising the following step:
contacting the compound according to any one of claims 48-61 with the drug according to any one of claims 1-47.

63. A pharmaceutical composition, comprising the antibody-drug conjugate according to any one of claims 1-47, and optionally a pharmaceutically acceptable carrier.

64. A method for modulating the tumor microenvironment in a subject, comprising the following step: administering to the subject the antibody-drug conjugate according to any one of claims 1-47, or the pharmaceutical composition according to claim 63.

65. A method for modulating the immune response in a subject, comprising the following step:
administering to the subject the antibody-drug conjugate according to any one of claims 1-47, or
the pharmaceutical composition according to claim 63.

66. Use of the antibody-drug conjugate according to any one of claims 1-47 or the pharmaceutical composition according to claim 63 in the preparation of a medicament, wherein the medicament can prevent and/or treat tumors.

67. The use according to claim 66, wherein the tumors include solid tumors and/or non-solid tumors.
